# EUROPÄISCHE PATENTSCHRIFT

(11) **EP 2 978 804 B1**
(45) Veröffentlichungstag und Bekanntmachung des Hinweises auf die Patenterteilung: **07.11.2018**
(21) Anmeldenummer: 14713798.8
(22) Anmeldetag: 24.03.2014
(51) Int. Cl.: C08K 5/00, C09K 21/10, C08K 5/3417

(54) **VERWENDUNG VON ORGANISCHEN OXYIMIDEN ALS FLAMMSCHUTZMITTEL FÜR KUNSTSTOFFE SOWIE FLAMMGESCHÜTZTE KUNSTSTOFFZUSAMMENSETZUNG UND HIERAUS HERGESTELLTE FORMTEILE**
USE OF ORGANIC OXY IMIDES AS FLAME RETARDANTS FOR PLASTICS AND FLAME-RETARDANT PLASTICS COMPOSITION AND MOULDINGS PRODUCED THEREFROM
UTILISATION D'OXIMIDES ORGANIQUES COMME AGENTS D'IGNIFUGATION DE MATIÈRES PLASTIQUES, COMPOSITION DE MATIÈRE PLASTIQUE IGNIFUGÉE ET PIÈCES MOULÉES FABRIQUÉES À PARTIR DE CETTE COMPOSITION

(30) Priorität: 25.03.2013 DE 102013005307
(43) Veröffentlichungstag der Anmeldung: 03.02.2016
(73) Patentinhaber: Fraunhofer-Gesellschaft zur Förderung der angewandten Forschung e.V., 80686 München (DE)
(72) Erfinder: PFAENDNER, Rudolf, 64668 Rimbach (DE); METZSCH-ZILLIGEN, Elke, 54597 Steffeln (DE); STEC, Maria, 64287 Darmstadt (DE)
(74) Vertreter: Pfenning, Meinig & Partner mbB
(86) Internationale Anmeldenummer: PCT/EP2014/055847
(87) Internationale Veröffentlichungsnummer: WO 2014/154636

(56) Entgegenhaltungen:
- US-A- 3 915 930
- US-A1- 2009 286 060
- SYDNEY M. SPATZ ET AL: "Some N-Substituted Tetrabromophthalimide Fire-Retardant Additives", INDUSTRIAL & ENGINEERING CHEMISTRY PRODUCT RESEARCH AND DEVELOPMENT, Bd. 8, Nr. 4, 1. Dezember 1969 (1969-12-01), Seiten 397-398, XP055121506, ISSN: 0196-4321, DOI: 10.1021/i360032a010

## Beschreibung

Die vorliegende Erfindung betrifft die Verwendung von organischen Oxyimiden als Flammschutzmittel für Kunststoffe. Gemäß der vorliegenden Erfindung wird ebenso eine flammgeschützte Kunststoffzusammensetzung angegeben, die ein Oxyimid als Flammschutzmittel beinhaltet. Zudem werden Formteile, hergestellt aus einer erfindungsgemäßen flammgeschützten Polymerzusammensetzung angegeben.

Die meisten Kunststoffe, wie z. B. Kunststoffe auf der Basis von Polyolefinen, Polystyrol, Polyamide, Polyurethane oder Polyestern, sind brennbar und vergleichsweise leicht entflammbar. Um das Brandrisiko von Kunststoffen in bestimmten Anwendungen zu vermindern oder auszuschließen, ist es deshalb zwingend erforderlich die Entflammbarkeit zu verringern und flammfeste oder flammgeschützte Kunststoffzusammensetzungen einzusetzen. Dazu werden in der Regel Flammschutzmittel dem Kunststoff beigegeben mit dem Ziel, das Entzünden für eine bestimmte Zeit zu verhindern oder die Brandausbreitung signifikant zu verzögern. Traditionelle Flammschutzmittel basieren auf chlor- und bromhaltigen Verbindungen (meist in Kombination mit Antimontrioxid), auf phosphorhaltigen, auf stickstoffhaltigen Verbindungen und/oder auf anorganischen Hydroxiden. In neuerer Zeit werden aus Umweltgründen und/oder toxikologischen Aspekten halogenfreie Flammschutzlösungen bevorzugt.

Zur Herstellung von flammgeschützten Kunststoffen gibt es eine Vielzahl von Flammschutzmitteln, die in der Regel substratspezifisch für ein bestimmtes Polymer und einen bestimmten Einsatzbereich entsprechend den dafür zugrundeliegenden Normen eingesetzt werden. Flammgeschützte Kunststoffe werden beispielsweise in Elektro- und Elektronikanwendungen, im Transport/Automobilbereich und im Bauwesen eingesetzt. Eine in den letzten Jahren entwickelte sehr wirksame Flammschutzmittelklasse auf Stickstoffbasis, vorzugsweise für Polyolefine, beruht auf Alkoxyaminen (WO 99/00450, WO 2008101845, WO 2011086114). Durch Spaltung der Alkoxyamine entstehen im Brandfall Radikale, die in den Abbauprozess des Polymeren eingreifen und damit die Flammschutzwirkung bewirken (C. R. Wilen, R. Pfaendner, J. Appl. Pol. Sci. 2013, R. Pfaendner, C.R. Chimie 9 (2006), 1338-1344). Die Alkoxyamine können auch vorteilhaft in synergistischen Kombinationen mit anderen Flammschutzmitteln eingesetzt werden (WO 02/074847, WO 03/016388, WO 2010026230, WO 2009080554, WO 2011003773, WO 2011117266). Weiterhin wurde gefunden, dass auch Hydroxylamin-Stabilisatoren eine synergistische Verbesserung der Wirkung von bromhaltigen, phosphorhaltigen und anorganischen Flammschutzmitteln bewirken können (WO 02/074847). US 2009/286060 offenbart eine Kunststoffzusammensetzung enthaltend 0.01-10 Gew.% eines Abbaubeschleunigers, zum Beispiel eines Halogen-freien organischen Oxyimids und bevorzugweise weitere Zusatzstoffe wie UV-Absorber, Lichtstabilisatoren, Metalldesaktivatoren oder Flammschutzmittel. Vielfache Auswahl Dokument SYDNEY M. SPATZ ET AL ("Some N-Substituted Tetrabromophthalimide Fire-Retardant Additives", INDUSTRIAL & ENGINEERING CHEMISTRY PRODUCT RESEARCH AND DEVELOPMENT, Bd. 8, Nr. 4, 1. Dezember 1969, Seiten 397-398) offenbart die Verwendung von halogenierten Imid-Derivaten als Flammschutzmittel für Kunststoffe, aber kein halogen-freies Oxyimid. US 3 915 930 offenbart die Verwendung von halogenierten Bisimide als Flammschutzmittel für Kunststoffe, aber kein halogen-freies Oxyimid. Die zuvor genannten aus dem Stand der Technik bekannten Flammschutzmittel erfüllen jedoch nicht alle heute an diese gestellten Anforderungen. So müssen diese beispielsweise nach wie vor in einer relativ hohen Konzentration bezüglich der zu schützenden Kunststoffzusammensetzung eingesetzt werden, was z.B. zu einer Verschlechterung der mechanischen Eigenschaften des Kunststoffs führen kann. Weitere Kriterien sind beispielsweise die thermische Stabilität, Verfärbung, Bildung von toxischen Gasen im Brandfall, einfache Zugänglichkeit im industriellen Maßstab u.a..

Aufgabe der vorliegenden Erfindung war es neue Flammschutzmittel und synergistische Flammschutzmittel-Komponenten zur Verfügung zu stellen, die in niedrigen Konzentrationen wirksam und vergleichsweise einfach zugänglich sind.

Diese Aufgabe wird hinsichtlich der Verwendung von organischen Oxyimiden als Flammschutzmittel für Kunststoffe gemäß den Merkmalen des Patentanspruchs 1, bezüglich einer flammgeschützten Kunststoffzusammensetzung mit den Merkmalen des Patentanspruchs 12, bezüglich eines Verfahrens zur Herstellung dieser Kunststoffzusammensetzung mit den Merkmalen des Patentanspruchs 15 sowie einem Formteil einem Lack oder einer Beschichtung, das aus einer flammgeschützten Polymerzusammensetzung herstellbar ist, mit den Merkmalen des Patentanspruchs 16 gelöst. Dabei stellen die jeweilig abhängigen Patentansprüche vorteilhafte Weiterbildungen dar.

Erfindungsgemäß wird somit die Verwendung von organischen Oxyimiden, enthaltend mindestens ein Strukturelement der nachfolgend abgebildeten Formel I 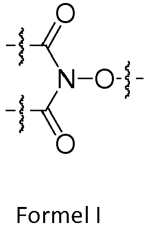 als Flammschutzmittel für Kunststoffe angegeben.

Die Formel I ist dabei so zu verstehen, dass das abgebildete Strukturelement in dem organischen Oxyimiden enthalten ist. Die erfindungsgemäß verwendeten Oxyimide sind dabei nicht gleichzusetzen mit Isocyanuraten bzw. hiervon abgeleiteten Verbindungen oder Verbindungsklassen. Das erfindungsgemäß verwendete Oxyimid ist dabei halogenfrei, d.h. die entsprechende Verbindung beinhaltet keine Halogenatome.

Es konnte gezeigt werden, dass die erfindungsgemäß verwendeten organischen Oxyimide wie voranstehend beschrieben gute Wirksamkeit aufweisen, um Kunststoffe flammhemmend auszustatten.

Es werden somit neue Flammschutzmittel und Flammschutzmittelzusammensetzungen für Polymere vorgeschlagen, die von der Wirkung und vom Preis/Leistungsverhältnis eine attraktive Alternative zu heute bekannten und verwendeten Zusammensetzungen darstellen.

Gemäß einer bevorzugten Ausführungsform der vorliegenden Erfindung werden Oxyimide, enthaltend mindestens ein Strukturelement der nachfolgenden Formel II 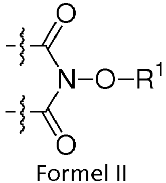 wobei R¹ für Wasserstoff oder einen gegebenenfalls substituierten Alkyl-, Cycloalkyl-, Aryl-, Heteroaryl- oder Acyl-Rest steht, als Flammschutzmittel verwendet.

Alternativ oder in Kombination mit der zuvor genannten bevorzugten Variante können ebenso verbrückte Oxyimide, enthaltend mindestens ein Strukturelement der nachfolgenden Formel III 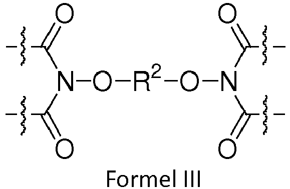 wobei R² für einen gegebenenfalls substituierten Alkylen-, Cycloalkylen-, Arylen-, Heteroarylen- oder verbrückenden Acyl-Rest steht, verwendet werden.

Gemäß einer bevorzugten Variante ist R² ausgewählt aus Resten der Gruppe bestehend aus
-(CH₂)ₙ- mit n = 1 bis 18, -CH(CH₃)-, -C(CH₃)₂-, -O-, -S-, -SO₂-, -NHCO-,-CO- sowie den nachfolgend abgebildeten Gruppen 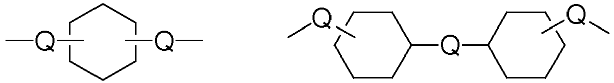 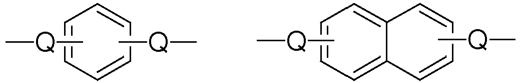 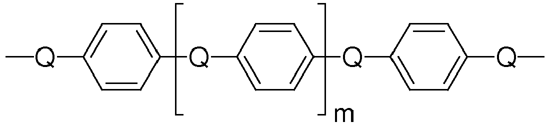 wobei
die in den zuvor abgebildeten Gruppen enthaltenen cycloaliphatischen oder aromatischen Ringsysteme unsubstituiert oder durch eine oder mehrere Alkyl- und/oder Alkoxygruppen substituiert sind,
- Q: bei jedem Auftreten gleich oder verschieden ist und ausgewählt ist aus der Gruppe bestehend aus einer chemischen Bindung sowie den Resten -(CH₂)ₙ- mit n = 1 bis 18, -CH(CH₃)-, -C(CH₃)₂-,-O-, -S-, -SO₂-, -NHCO-,
-CO-, und
- m: 0 oder 1 bis 18 ist.

Besonders bevorzugt sind die Reste R² dabei durch die nachfolgend abgebildeten Strukturelemente wiedergegeben, wobei Q die oben angegebene Bedeutung aufweist:

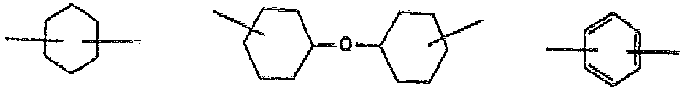

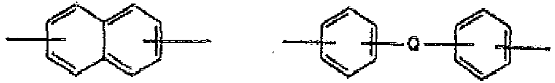

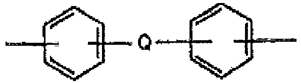

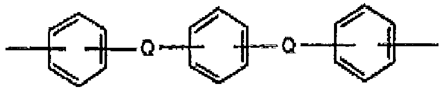

Insbesondere können die Reste R² dabei durch die nachfolgenden Strukturelemente gegeben sein:

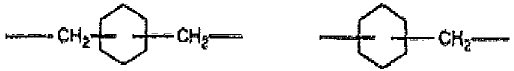

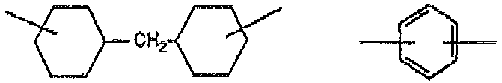

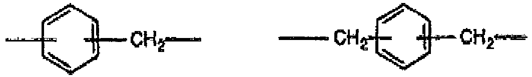

Gemäß einer besonders bevorzugten Ausführungsform weist das organische Oxyimid eine der nachfolgenden Strukturformel auf: 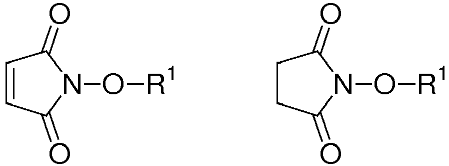 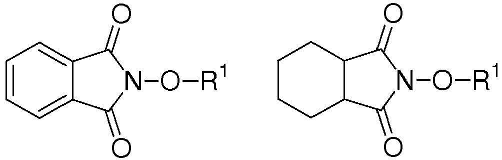 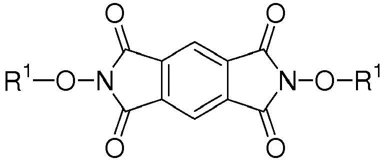 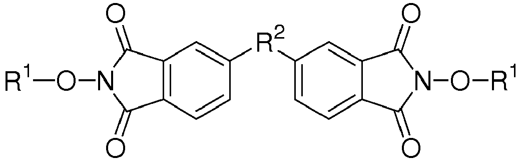 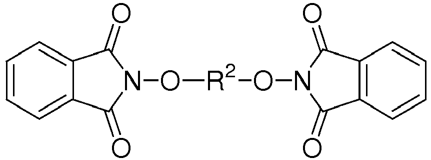 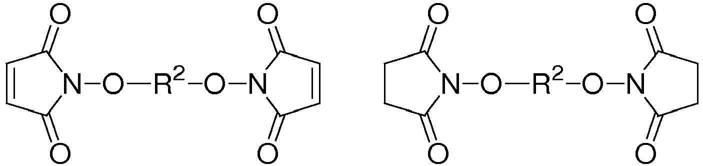 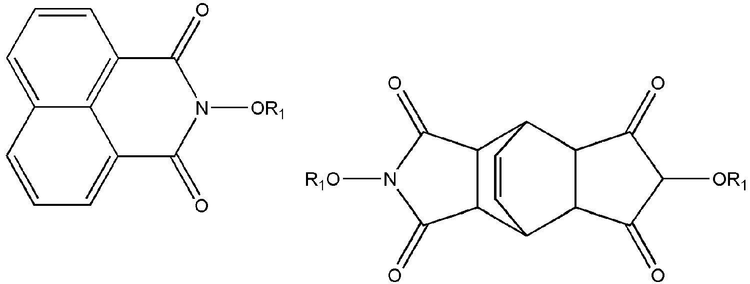 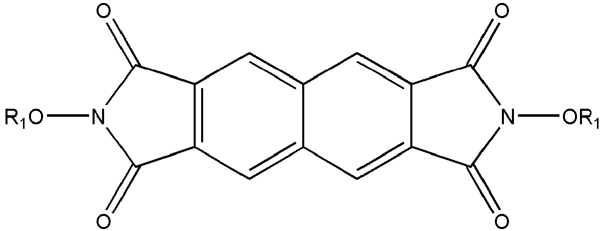 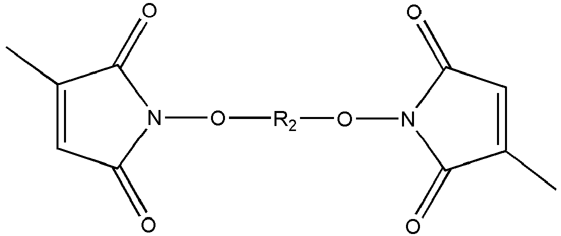 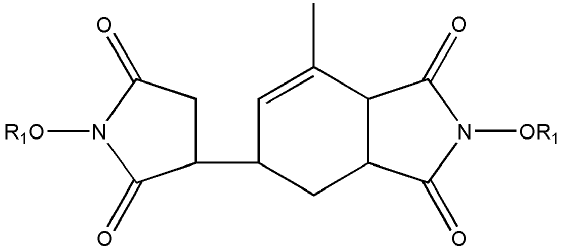 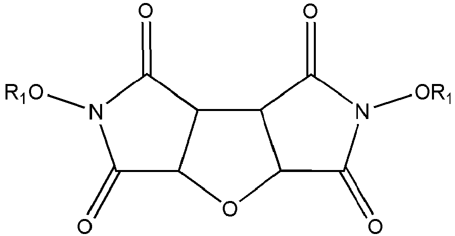 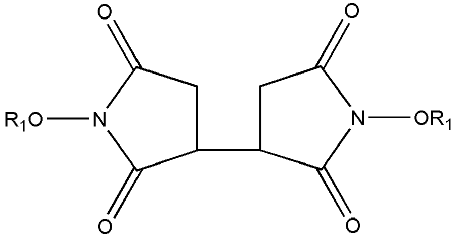 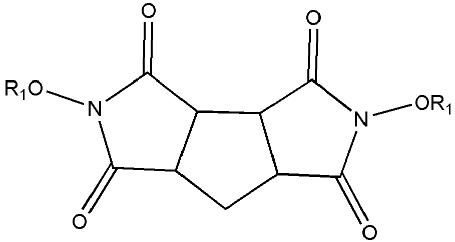 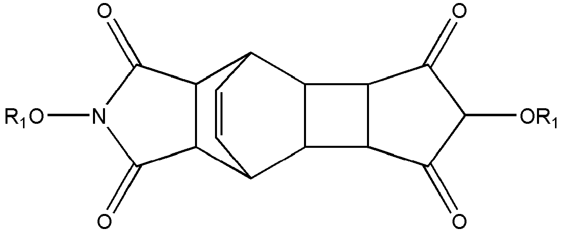 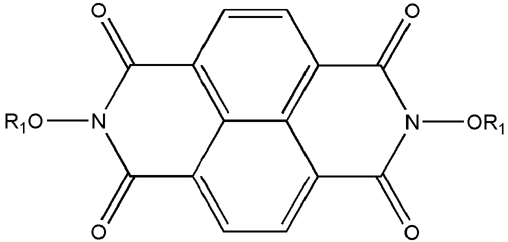 wobei jeweils R¹ und R² die oben angegebene Bedeutung aufweisen.

Ein besonders bevorzugter Rest R¹ ist dabei Wasserstoff oder ein Acylrest.

Insbesondere eignen sich für die Zwecke der vorliegenden Erfindung die nachfolgend abgebildeten Verbindungen mit R¹ = H 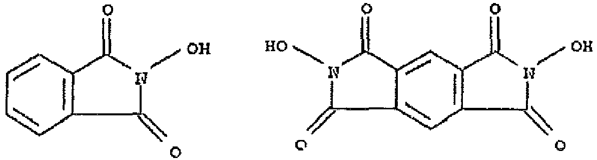 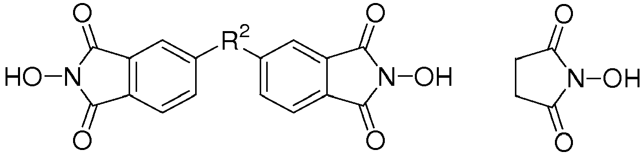 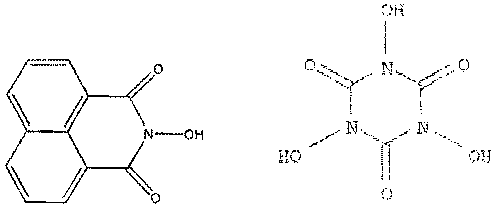 wobei R² die oben angegebene Bedeutung aufweist.

Insbesondere eignen sich für die Zwecke der vorliegenden Erfindung auch die nachfolgend abgebildeten Verbindungen mit R¹ = Acyl.

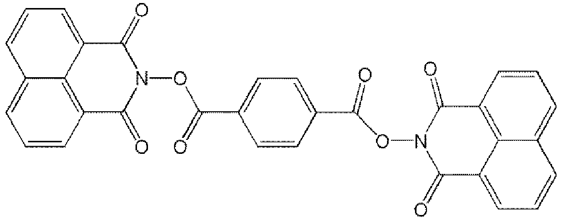

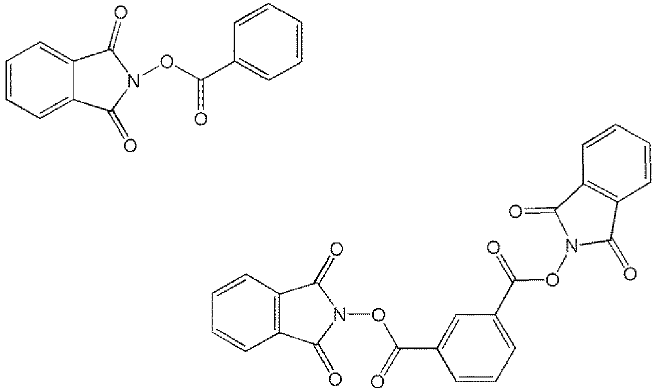

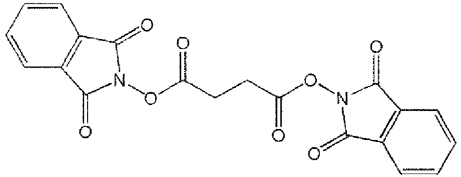

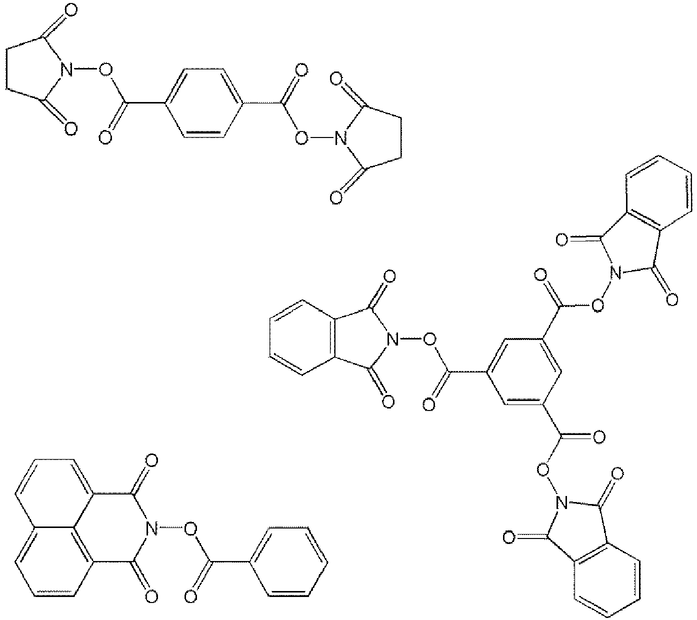

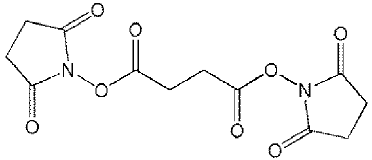

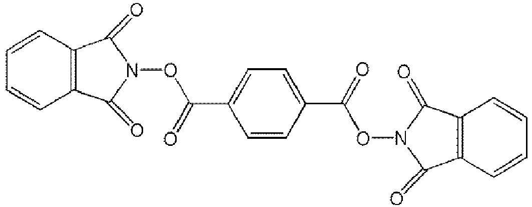

Die oben angegebenen Verbindungen mit R1 = H sind beispielsweise durch Reaktion von kommerziell erhätlichen Di- oder Tetracarbonsäureanhydriden mit Hydroxylamin in Form des Hydrochlorids zugänglich bzw. dann mit R1 = H oder R2 = Acyl durch nachfolgende Veresterung der erhaltenen Hydroxylamine mit beispielsweise einem Säurechlorid oder durch Umesterung aus den entsprechenden Säurestern mit z.B. leichtflüchtigen Alkoholen wie z.B. den Methylestern.

Weiterhin können diese Hydroxylaminderivate auch in Form von linearen Oligomeren oder Polymeren vorliegen, indem z.B. difunktionelle Hydroxylamine (R1 = H) z.B. mit difunktionellen Säurechloriden umgesetzt und dann die entsprechenden Polyester erhalten werden. In analoger Weise können aus den difunktionellen Hydroxylaminen (R1 = H) und Diisocyanaten die entsprechenden linearen Polyurethane hergestellt werden. Die Synthesen dieser Polymeren ist beispielsweise in H. Imajo et al. J. Pol. Sci: Pol. Chem. 18, 2189-2196 (1980) oder in H. Imajo et al. J. Pol. Sci.: Pol. Chem. 19, 1855-1861 (1981) näher beschrieben.

Wenn gewünscht können diese Polymeren auch in Form von verzweigten oder vernetzten Strukturen synthetisiert werden, indem teilweise oder vollständig eine difunktionelle Komponente durch eine tri-, tetra-, oder höher funktionalisierte Verbindung ersetzt wird, z.B. eine trifunktionelle Säurekomponente oder ein trifunktionelles Hydroxylamin.

Weiterhin ist es möglich und für den Fachmann ersichtlich, dass Copolymere erfindungsgemäß synthetisiert werden können, indem z.B. ein difunktionelles Hydroxylimid teilweise durch eine Diolverbindung wie z.B. Ethylenglycol, Butandiol-1-4, Hexandiol-1,6, Hydrochinon, 4,4'-Dihydroxybiphenyl ersetzt wird. Dementsprechend werden z.B. Copolyester oder Copolyurethane zugänglich. Darüberhinaus sind durch teilweisen Austausch der difunktionellen Hydroxylimide durch difuntionelle Verbindungen anderer Strukturen als Diole, wie z.B. Diamine Copolymere gemischter Strukturen wie z.B. Polyesteramide erhältlich.

Gemäß der vorliegenden Erfindung können die erfindungsgemäß verwendeten organischen Oxyimide bevorzugt für die folgend dargestellten Kunststoffe, besonders bevorzugt in thermoplastischen, elastomeren oder duroplastischen Kunststoffen, insbesondere thermoplastischen Polymeren, eingesetzt werden:
a) Polymere aus Olefinen oder Diolefinen wie z.B. Polyethylen (LDPE, LLDPE, VLDPE, ULDPE, MDPE, HDPE, UHMWPE), Metallocen-PE (m-PE), Polypropylen, Polyisobutylen, Poly-4-methyl-penten-1, Polybutadien, Polyisopren, Polycycloocten, Polyalkylen-Kohlenmonoxid-Copolymere, sowie Copolymere in Form von statistischen oder Blockstrukturen wie z.B. Polypropylen-Polyethylen (EP), EPM oder EPDM, EthylenVinylacetat (EVA), Ethylen-Acrylester, wie z.B. Ethylen-Butylacrylat, Ethylen-Acrylsäure und deren Salze (lonomere), sowie Terpolymere wie z.B. Ethylen-Acrylsäure-Glycidylacrylat, Pfropfpolymere wie z.B. Polypropylen-g-Maleinsäureanhydrid, Polypropylen-g-Acrylsäure, Polyethylen-g-Acrylsäure,
b) Polystyrol, Polymethylstyrol, Polyvinylnaphthalin, Styrol-Butadien (SB), Styrol-Butadien-Styrol (SBS), Styrol-Ethylen-Butylen-Styrol (SEBS), Styrol-Ethylen-Propylen-Styrol, Styrolisopren, Styrol-Isopren-Styrol (SIS), Styrol-butadienacrylnitril (ABS), Styrol-acrylnitril-acrylat (ASA), Styrol-Ethylen, Styrol-Maleinsäureanhydrid-Polymere einschl. entsprechender Pfropfcopolymere wie z.B. Styrol auf Butadien, Maleinsäureanhydrid auf SBS oder SEBS, sowie Pfropfcopolymere aus Methylmethacrylat, Styrol-Butadien und ABS (MABS),
c) halogenenthaltende Polymere wie z.B. Polyvinylchlorid (PVC), Polychloropren und Polyvinylidenchlorid (PVDC), Copolymere aus Vinylchlorid und Vinylidenchlorid oder aus Vinylchlorid und Vinylacetat, chloriertes Polyethylen, Polyvinylidenfluorid,
d) Polymere von ungesättigten Estern wie z.B. Polyacrylate und Polymethacrylate wie Polymethylmethacrylat (PMMA), Polybutylacrylat, Polylaurylacrylat, Polystearylacrylat, Polyacrylnitril, Polyacrylamide, Copolymere wie z.B. Polyacrylnitril-Polyalkylacrylat,
e) Polymere aus ungesättigten Alkoholen und Derivaten, wie z.B. Polyvinylalkohol, Polyvinylacetat, Polyvinylbutyral,
f) Polyacetale, wie z.B. Polyoxymethylen POM) oder Copolymere mit z.B. Butanal,
g) Polyphenylenoxide und Blends mit Polystyrol oder Polyamiden,
h) Polymere von cyclischen Ethern wie z.B. Polyethylenglycol, Polypropylenglycol, Polyethylenoxid, Polypropylenoxid,
i) Polyurethane, aus hydroxyterminierten Polyethern oder Polyestern und aromatischen oder aliphatischen Isocyanaten insbesondere lineare Polyurethane, Polyharnstoffe,
j) Polyamide wie z.B. Polyamid-6, 6.6, 6.10, 4.6, 4.10, 6.12, 12.12, Polyamid 11, Polyamid 12 sowie (teil-)aromatische Polyamide wie z.B. Polyphthalamide, z.B. hergestellt aus Terephthalsäure und/oder Isophthalsäure und aliphatischen Diaminen oder aus aliphatischen Dicarbonsäuren wie z.B. Adipinsäure oder Sebazinsäure und aromatischen Diaminen wie z.B. 1,4- oder 1,3-Diaminobenzol,
k) Polyimide, Polyamid-imide, Polyetherimide, Polyesterimide, Poly(ether)ketone, Polysulfone, Polyethersulfone, Polyarylsulfone, Polyphenylensulfid, Polybenzimidazole, Polyhydantoine,
l) Polyester aus aliphatischen oder aromatischen Dicarbonsäuren und Diolen oder aus Hydroxy-Carbonsäuren wie z.B. Polyethylenterephthalat (PET), Polybutylenterephthalat (PBT), Polypropylenterephthalat, Polyethylennaphthylat, Poly-1,4-dimethylolcyclohexanterephthalat, Polyhydroxybenzoat, Polyhydroxynaphthalat, Polymilchsäure,
m) Polycarbonate, Polyestercarbonate, sowie Blends wie z.B. PC/ABS, PC/PBT, PC/PET/PBT,
n) Cellulosederivate wie z.B. Cellulosenitrat, Celluloseacetat, Cellulosepropionat, Cellulosebutyrat
o) nicht-thermoplastischen oder duroplastischen Kunststoffen
p) sowie Mischungen, Kombinationen oder Blends aus zwei oder mehr der zuvor genannten Polymere.

Sofern es sich bei den unter a) bis o) angegebenen Polymeren um Copolymere handelt, können diese in Form von statistischen ("random"), Block- oder "tapered" Strukturen vorliegen.

Sofern es sich bei den unter a) bis o) angegebenen Polymeren um stereoreguläre Polymere handelt, können diese in Form von isotaktischen, stereotaktischen, aber auch ataktischen Formen vorliegen.

Weiterhin können die unter a) bis o) angegebenen Polymere sowohl amorphe als auch (teil-)kristalline Morphologien aufweisen.

Ggf. können die unter a) genannten Polyolefine auch vernetzt vorliegen, z.B. vernetztes Polyethylen, das dann als X-PE bezeichnet wird. Die unter a) genannten Polyolefine können beliebige Stereostrukturen aufweisen, d.h. isotaktisch, syndiotaktisch oder ataktisch oder in Stereoblockstrukturen vorliegen.

Ganz besonders bevorzugt werden die erfindungsgemäß verwendeten organischen Oxyimide für Polyolefine, insbesondere für die unter a) genannten Polyolefine, eingesetzt.

Darüber hinaus können die vorliegenden Flammschutzmittel in den folgenden duromeren, nicht-thermoplastischen Kunststoffen Verwendung finden:
q) Epoxidharze, bestehend aus di- oder polyfunktionellen Epoxidverbindungen in Kombination mit z.B. aminischen, anhydridischen oder katalytisch wirkenden Härtern,
r) Phenolharze wie z.B. Phenol-Formaldehyd-Harze, Harnstoff-Formaldehyd-Harze, Melamin-Formaldehydharze,
s) ungesättigte Polyesterharze,
t) Silikone,
u) Polyurethane als Reaktionsprodukte aus di- oder polyfunktionellen Isocyanaten und Polyolen, Polyharnstoffe,
v) Alkydharze, Allylharze.

Ganz besonders bevorzugt werden die erfindungsgemäßen Flammschutzmittel bei Polyolefinen, vorzugsweise Polypropylen und/oder Polyethylen und deren Copolymeren und Blends eingesetzt.

Insbesondere kann das erfindungsgemäß eingesetzte Oxyimid in Kombination mit mindestens einem weiteren Flammschutzmittel verwendet werden, wodurch sich synergistische Effekte ergeben. Das mindestens eine weitere Flammschutzmittel ist dabei bevorzugt ausgewählt aus der Gruppe bestehend aus
a) anorganischen Flammschutzmitteln wie z.B. Al(OH)₃, Mg(OH)₂, AIO(OH), MgCO₃, Schichtsilikate wie z.B. Montmorillonit, nicht oder organisch modifiziert, Doppelsalze, wie z.B. Mg-Al-Silikate, POSS-(Polyhedral Oligomeric Silsesquioxane) Verbindungen, Huntit, Hydromagnesit oder Halloysit sowie Sb₂O₃, Sb₂O₅, MoO₃, Zinkstannat, Zinkhydroxystannat,
b) stickstoffhaltigen Flammschutzmitteln wie z.B. Melamin, Melem, Melam, Melon, Melaminderivate, Melaminkondensationsprodukte oder Melaminsalze, Benzoguanamin, Polyisocyanurate, Allantoin, Phosphacene, insbesondere Melamincyanurat, Melaminphosphat, Dimelaminphosphat, Melaminpyrophosphat, Melaminpolyphosphat, Melamin-Metall-Phosphate wie z.B. Melaminaluminumphosphat, Melaminzinkphosphat, Melaminmagnesiumphopsphat, sowie die entsprechenden Pyrophosphate und Polyphosphate, Poly-[2,4-(piperazin-1,4-yl)-6-(morpholin-4-yl)-1,3,5-triazin], Ammoniumpolyphosphat, Melaminborat, Melaminhydrobromid,
c) Radikalbildnern,
d) Phosphorhaltigen Flammschutzmitteln wie z.B. roter Phosphor, Phosphate wie z.B. Resorcindiphosphat, Bisphenol-A-diphosphat und ihre Oligomere, Triphenylphosphat, Ethylendiamindiphosphat, Phosphinate wie z.B. Salze der hypophosphorigen Säure und Ihrer Derivate wie Diethylaluminiumphosphinat oder Aluminiumphosphinat, Aluminiumphosphit, Aluminiumphosphonat, Phosphonatester, oligomere und polymere Derivate der Methanphosphonsäure, 9,10-Dihydro-9-oxa-10-phosphorylphenanthren-10-oxid (DOPO) und deren substituierte Verbindungen,
e) Halogenhaltigen Flammschutzmitteln auf Chlor- und Brombasis wie z.B. polybrominierte Diphenyloxide, wie z.B. Decabromdiphenyloxid,Tris(3-bromo-2,2-bis(bromo-methyl)propyl-phosphat, Tris(tribromneopentyl)phosphat, Tetrabromphthalsäure, 1,2-Bis(tribromphenoxy)ethan, Hexabromcyclododecan, bromiertes Diphenylethan, Tris-(2,3-dibrompropyl)isocyanurat, Ethylen-bis(tetrabromophthalimid), Tetrabromo-bisphenol A, bromiertes Polystyrol, bromiertes Polybutadien bzw, Polystyrol-bromiertes Polybutadien-Copolymere, bromiertes Epoxidharz, Polypentabrombenzylacrylat, ggf. in Kombination mit Sb₂O₃ und/oder Sb₂O₅,
f) Boraten wie z.B. Zinkborat oder Calciumborat,
g) Antidrip-Mitteln wie z.B. Polytetrafluorethylen,
h) siliciumhaltigen Verbindungen wie z.B. Polyphenylsiloxane.

Bei den unter b) genannten halogenhaltigen Flammschutzmitteln handelt es sich häufig um kommerzielle Produkte, die z.B. von den Firmen Albemarle, Chemtura/Great Lakes oder ICL im Handel erhältlich sind.

Insbesondere bei Kombinationen des erfindungsgemäß verwendeten Oxyimid mit mindestens einem Radikalbildner als weiteres Flammschutzmittel ergeben sich synergistische Effekte.

Radikalbildner im Sinne der vorliegenden Erfindung sind Verbindungen, die durch thermische und Licht-induzierte Spaltung Radikale erzeugen können. Geeignete Radikalbildner für die hier vorliegenden Anwendungen sind solche, die für die Kunststoff- oder Beschichtungs-Verarbeitungsprozesse eine ausreichende thermische Stabilität aufweisen, d.h. bei der Verarbeitung noch keine oder nur sehr geringe Mengen an Radikalen bilden und erst bei höheren Temperaturen, wie sie erst im Brandfall auftreten, spontan Radikale erzeugen. Die jeweiligen Verarbeitungsprozesse und Temperaturen für Beschichtungen und Kunststoff-Verarbeitungsprozesse sind dem Fachmann bekannt. Kunststoff-Verarbeitungsprozesse und dazugehörige Temperaturen können aber auch der Fachliteratur entnommen werden wie z.B. H. Domininghaus, P. Elsner, P. Eyerer. T. Hirth, Kunststoffe , 8. Auflage, Springer 2012.

Der Radikalbildner ist dabei bevorzugt ausgewählt aus der Gruppe bestehend aus N-Alkoxyaminen, -C-C- Radikalbildnern, Radikalbildnern mit Azogruppen (-N=N-), Radikalbildnern mit Hydrazingruppen (-NH-HN-), Radikalbildnern mit Hydrazongruppen (>C=N-NH-), Radikalbildnern mit Azingruppen (>C=N-N=C<), Radikalbildnern mit Triazengruppen (-N=N-N<), Radikalbildnern mit Disulfid- bzw. Polysulfidgrupen (-S-S-), Radikalbildnern mit Thiolgruppen (-S-H), Thiuramsulfid, Dithiocarbamaten, Mercaptobenzthiazol und Sulfenamiden.

Der Radikalbildner ist dabei besonders bevorzugt ausgewählt aus der Gruppe bestehend aus
a) N-Alkoxyaminen gemäß der nachfolgend abgebildeten Strukturformel 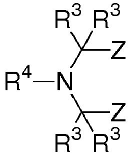 wobei
   - R³: für Wasserstoff oder einen gegebenenfalls substituierter Alkyl-, Cycloalkyl-, Aryl-, Heteroaryl- oder Acyl-Rest steht, insbesondere ein C1 bis C4- Alkylrest ist,
   - R⁴: für einen Alkoxy-, Aryloxy-, Cycloalkoxy-, Aralkoxy- oder Acyloxy-Rest steht,
   - Z: für Wasserstoff oder einen gegebenenfalls substituierter Alkyl-, Cycloalkyl-, Aryl-, Heteroaryl- oder Acyl-Rest steht, wobei die beiden Reste Z auch einen geschlossenen Ring bilden können, der ggf. durch Ester-, Ether-, Amin, Amid, Carboxy- oder Urethangruppen substituiert sein kann,
b) Azo-Verbindungen gemäß der nachfolgend abgebildeten Strukturformeln 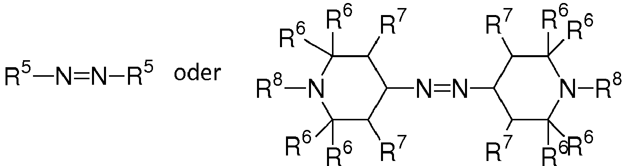 wobei
   - R⁵: einen Alkly-, Cycloalkyl- oder Arylrest bedeutet,
   - R⁶: bei jedem Auftreten gleich oder verschieden ist und einen linearen oder verzweigten Alkylrest bedeutet,
   - R⁷: bei jedem Auftreten gleich oder verschieden ist und Wasserstoff oder einen linearen oder verzweigten Alkylrest bedeutet, und
   - R⁸: bei jedem Auftreten gleich oder verschieden ist und einen Alkyl, Alkoxy-, Aryloxy- Cycloalkyloxy-, Aralkoxy oder Acyloxyrest bedeutet,
c) Dicumyl gemäß der nachfolgend abgebildeten Strukturformel 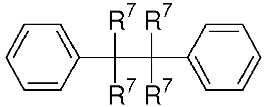 wobei R⁷ die zuvor angegebene Bedeutung aufweist, bevorzugt Methyl ist,
d) und/oder Polycumyl gemäß der nachfolgend abgebildeten Strukturformel 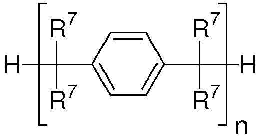 wobei R⁷ die zuvor angegebene Bedeutung aufweist, bevorzugt Methyl ist, und 2 < n < 100.

Typische Beispiele für die zuvor genannten N-Alkoxyamine der angegebenen Struktur sind dabei:
1-Cyclohexyloxy-2,2,6,6-Tetramethyl-4-Octadecylaminopiperidin; Bis(1-Octyloxy-2,2,6,6-Tetramethylpiperidin-4-yl) Sebacat; 2,4-Bis[(1-Cyclohexyloxy-2,2,6,6-Tetramethylpiperidin-4-yl)Butylamino]-6-(2-Hydroxyethylamino-S-Triazin; Bis(1-Cyclohexyloxy-2,2,6,6-Tetramethylpiperidin-4-yl) Adipat; 2,4-Bis[(1-Cyclohexyloxy-2,2,6,6-Tetramethylpiperidin-4-yl)Butylamino]-6-Chloro-S-Triazin; 1-(2-Hydroxy-2-Methylpropoxy)-4-Hydroxy-2,2,6,6-Tetramethylpiperidin; 1-(2-Hydroxy-2-Methylpropoxy)-4-Oxo-2,2,6,6-Tetramethylpiperidin; 1-(2-Hydroxy-2-Methylpropoxy)-4-Octadecanoyloxy-2,2,6,6-Tetramethylpiperidin; Bis(1-(2-Hydroxy-2-Methylpropoxy)-2,2,6,6-Tetramethylpiperidin-4-yl) Sebacat; Bis(1-(2-Hydroxy-2-Methylpropoxy)-2,2,6,6-Tetramethylpiperidin-4-yl) Adipat; 2,4-Bis{N-[1-(2-Hydroxy-2-Methylpropoxy)-2,2,6,6-Tetramethylpiperidin-4-yl]-N-Butylamino}-6-(2-Hydroxyethylamino)-S-Triazin); 4-Piperidinol, 2,2,6,6-tetramethyl-1-(undecyloxy)-, 4,4'-carbonat; das Reaktionsprodukt von 2,4-Bis[(1-Cyclohexyloxy-2,2,6,6-Tetramethylpiperidin-4-yl)Butylamino]-6-Chloro-S-Triazin mit N,N'-Bis(3-Aminopropylethylendiamin); die Oligomer-Verbindung, welche das Kondensationsprodukt ist von 4,4'-Hexamethylen-Bis(Amino-2,2,6,6-Tetramethylpiperidin) und 2,4-Dichloro-6-[(1-Cyclohexyloxy-2,2,6,6-Tetramethyl-4-yl)Butylamino]-S-Triazin, an den Enden verschlossen mit 2-Chloro-4,6-Bis(Dibutylamino)-S-Triazin; aliphatische Hydroxylamin wie z.B. Disterarylhydroxylamin; sowie Verbindungen der Formel 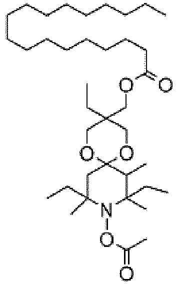 oder 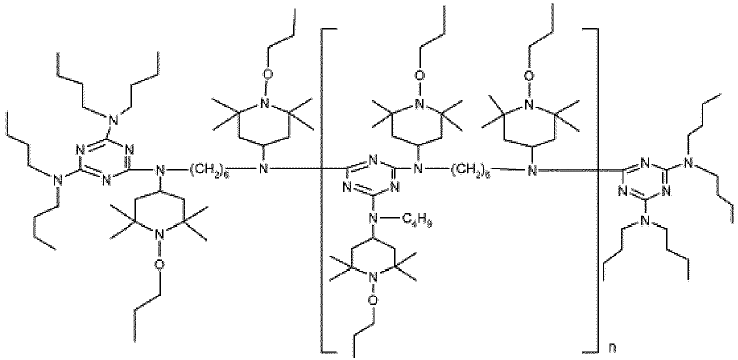 in denen n = 1-15 ist.

Die oben genannten Verbindungen sind teilweise kommerzielle Produkte und werden unter den folgenden Handelsnamen gehandelt: FLAMESTAB NOR 116 (RTM), TINUVIN NOR 371 (RTM), IRGATEC CR 76 (RTM) von BASF SE, Hostavin NOW (RTM) von Clariant oder ADK Stab LA 81 (RTM) von Adeka. Dicumyl und Polycumyl sind Handelsprodukte, die z.B. von United Initiators erhältlich sind.

Das mindestens eine weitere Flammschutzmittel kann insbesondere auch ein phosphorhaltiges Flammschutzmittel sein. Bevorzugte phosphorhaltige Flammschutzmittel sind dabei Phosphinate der folgenden Strukturen: 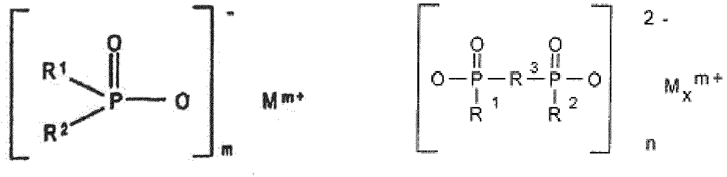 wobei bevorzugt R1 und R2 identisch oder verschieden sind und ausgewählt sind aus linearem oder verzweigtem C1-C6-Alkyl und/oder Aryl; M ausgewählt ist aus der Gruppe bestehend aus Mg, Ca, Al, Sb, Sn, Ge, Ti, Fe, Zr, Ce, Bi, Sr, Mn, Li, Na, K, Zn und/oder einem protonierten Stickstoff-Base, vorzugsweise Calcium-Ionen, Magnesium-Ionen, Aluminium-Ionen, und/oder Zink-Ionen; und m = 1-4, bevorzugt 2 oder 3, ist; n = 1-4, bevorzugt 1 oder 3, ist; x = 1-4, bevorzugt 1 oder 2, ist. In einer besonders bevorzugten Ausführungsform ist R₁ = Alkyl, R₂ = Alkyl und M = Al oder Zn.

Ein besonders bevorzugtes Beispiel für ein erfindungsgemäßes Phosphinat sind die kommerziell erhältlichen Produkte Exolit OP (RTM) von Clariant SE.

Weitere bevorzugte phosphorhaltige Flammschutzmittel sind Metallsalze der hypophosphorigen Säure mit einer Struktur gemäß der Formel 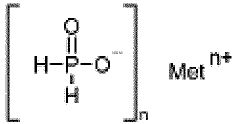 wobei Met ein Metall ist, ausgewählt aus den Gruppen I, II, III und IV des Periodensystems der Elemente, und n eine Zahl von 1 bis 4 ist, die der Ladung des entsprechenden Metall-Ions Met entspricht. Metⁿ⁺ ist beispielsweise Na⁺, Ca²⁺, Mg²⁺, Zn²⁺, Ti⁴⁺ oder Al³⁺, wobei Ca²⁺, Zn²⁺ und Al³⁺ besonders bevorzugt sind.

Die oben genannten Salze der hypophosphorigen Säure sind teilweise kommerziell erhältlich z.B. unter der Bezeichnung Phoslite (RTM) von Italmatch Chemicals.

Eine weitere bevorzugte Gruppe von Phosphorhaltigen Flammschutzmitteln sind Phosphonate oder Phosphonsäurediarylester einer Struktur gemäß der folgenden Formel: 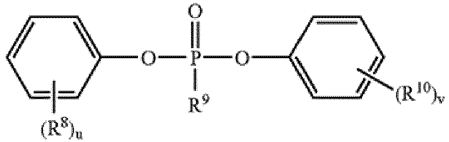 wobei R₈ und R₁₀ = H, Alkyl, vorzugsweise C1-C4-Alkyl sind, R₉ = C1-C4-Alkyl, u = 1-5 ist und v = 1-5 ist.

Entsprechende Strukturen können auch in der Form von Phosphonat-Oligomeren, Polymeren und Co-Polymeren vorliegen. Linear oder verzweigte Phosphonat-Oligomere und Polymere sind aus dem Stand der Technik bekannt. Für verzweigte Phosphonat-Oligomere und Polymere wird auf die US-Patente US 2 716 101, US 3 326 852, US 4 328 174, US 4 331 614, US 4 374 971, US 4 415 719, US 5 216 113, US 5 334 692, US 3 442 854, US 6 291 630 B1 US 6 861 499 B2 und US 7816486 B2 verwiesen. Für Phosphonat-Oligomere wird auf die US-Patentanmeldungen US 2005/0020800 A1, US 2007/0219295 A1 und US 2008/0045673 A1 verwiesen. In Bezug auf lineare Phosphonat-Oligomere und Polymere wird auf die US-Patent-Dokumente US 3 946 093, US 3 919 363, US 6 288 210 B1, US 2 682 522 und US 2 891 915 verwiesen.

Phophonate sind beispielsweise unter dem Handelsnamen Nofia (RTM) von FRX Polymers erhältlich.

Eine weitere bevorzugte Gruppe von phosphorhaltigen Flammschutzmitteln sind Verbindungen auf Basis von Oxaphosphorinoxid und deren Derivate mit beispielsweise den folgenden Strukturen: 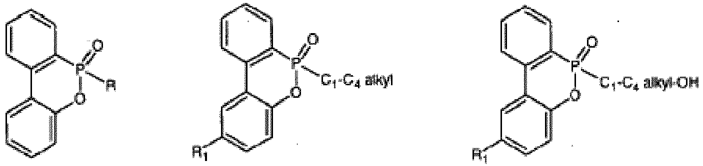 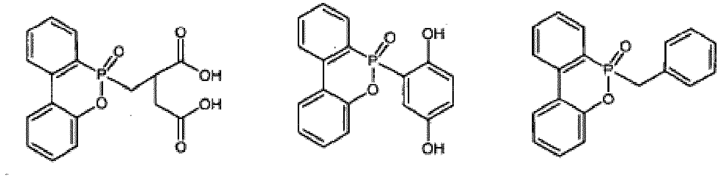 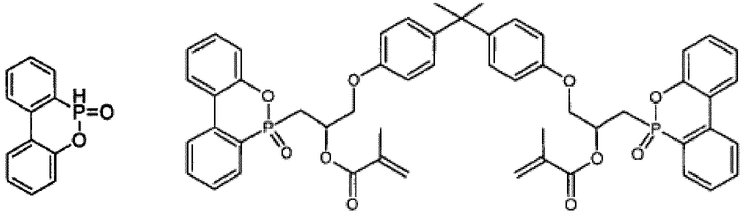 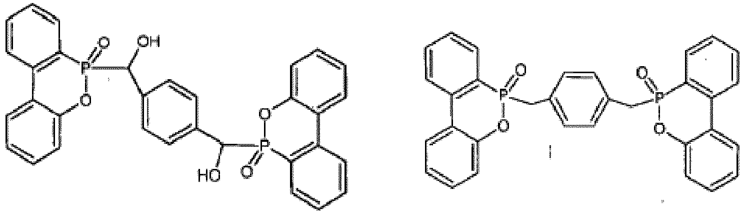 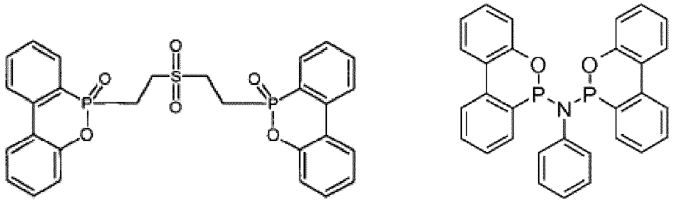 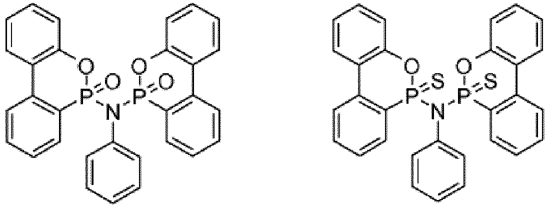 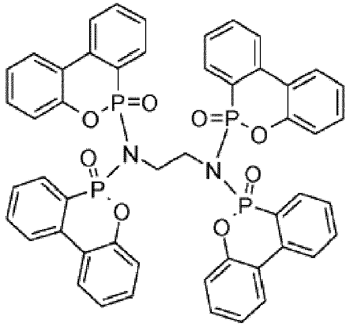 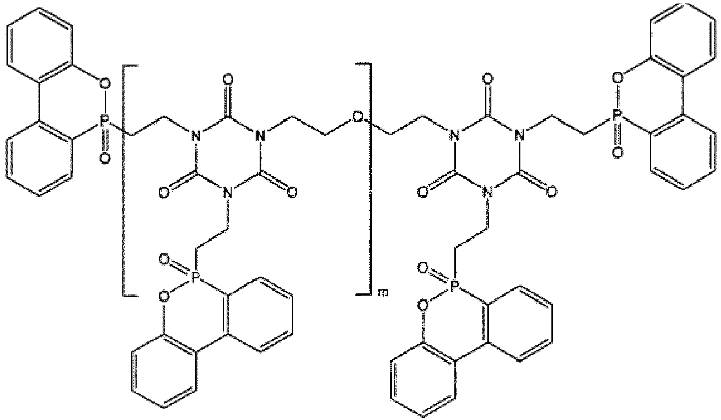 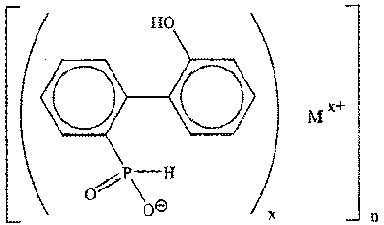 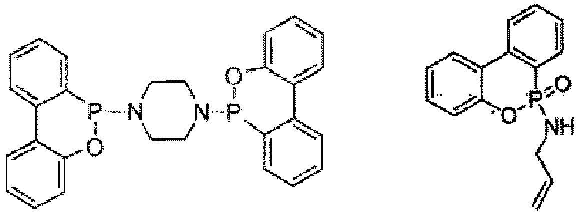 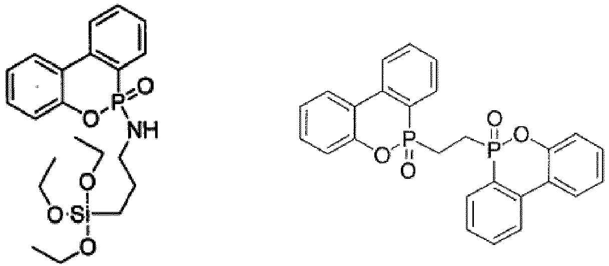 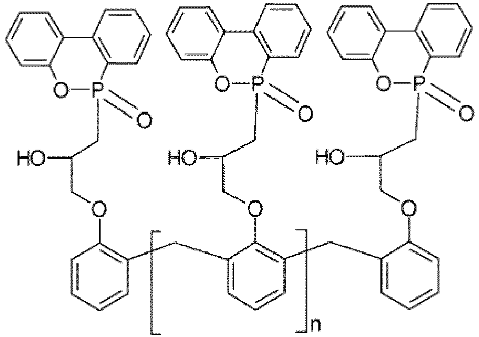 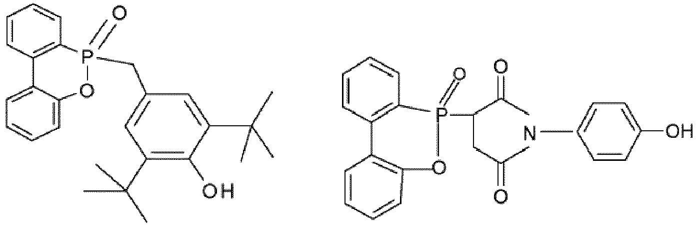 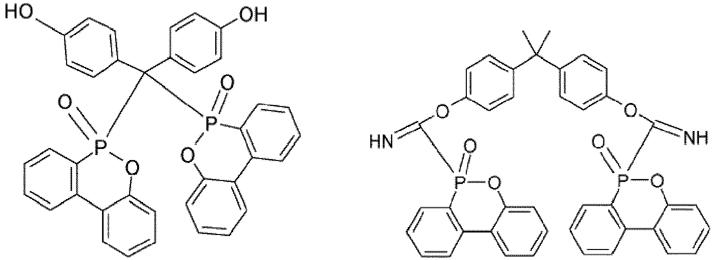 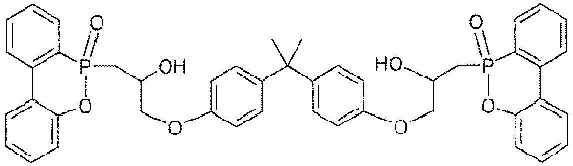 wobei M ein Metall ist, ausgewählt aus der zweiten, dritten, zwölften oder dreizehnten Gruppe des Periodensystems der Elemente ist, x = 2 oder 3 ist, n ≥ 10 ist, m= 0-25ist, R = H, Halogen oder ein aliphatischer oder aromatischer Rest mit 1-32 C-Atomen ist und R₁ = H, C1-C6-Alkyl ist.

Produkte auf der Basis von Oxophosphorinoxid sind beispielsweise unter dem Handelsnamen Ukanol (RTM) von Schill und Seilacher GmbH im Handel. Weitere Verbindungen können beispielsweise gemäß der Patentschriften WO 2013020696, WO 2010135398, WO03070736, WO2006084488, WO 2006084489, WO2011000019, WO2013068437, WO2013072295 hergestellt werden.

Weitere synergistische phosphorhaltige Flammschutzmittel sind zyklische Phosphonate einer Struktur gemäß einer der folgenden Formeln: 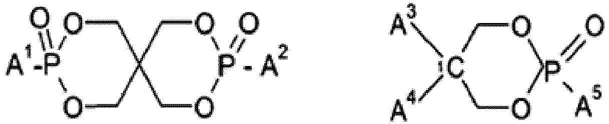 wobei A¹ und A² unabhängig voneinander eine substituierte oder nicht substituierte, geradkettige oder verzweigte Alkylgruppe mit 1 bis 4 Kohlenstoffatomen, substituiertes oder nicht substituiertes Benzyl, substituiertes oder nicht substituiertes Phenyl, substituiertes oder nicht substituiertes Naphthyl darstellten und wobei A³ und A⁴ unabhängig voneinander Methyl oder Ethyl sind und A⁵ eine geradkettige oder verzweigte Alkylgruppe mit 1 bis 4 Kohlenstoffatomen oder eine Phenyl- oder Benzylgruppe ist, die jeweils bis zu 3 Methylgruppen aufweisen kann, ist.

Zyklische Phosphonate sind beispielsweise von der Fa. Thor GmbH unter dem Handelsnamen Aflammit (RTM) im Handel oder können gemäß EP 2450401 hergestellt werden.

Das mindestens eine weitere Flammschutzmittel kann insbesondere auch ein Stickstoffhaltiges Flammschutzmittel sein. Bevorzugte Stickstoffhaltige Flammschutzmittel sind Melaminpolyphosphat, Melamincyanurat, Melamin-Metall-Phosphate, Poly-[2,4-(piperazin-1,4-yl)-6-(morpholin-4-yl)-1,3,5-triazin] und Ammoniumpolyphosphat. Diese Verbindungen sind kommerzielle Produkte und unter den Handelsnamen Melapur (RTM) von BASF SE, Budit (RTM) von Budenheim Chemische Fabrik, Exolit (RTM) von Clariant, Safire (RTM) von Floridienne oder MCA PPM Triazine von MCA Technologies GmbH erhältlich.

Im Falle einer kombinatorischen Verwendung des erfindungsgemäß verwendeten Oxyimids mit mindestens einem weiteren Flammschutzmittel ist es bevorzugt, wenn die zuvor genannten Verbindungen in einem Gewichtsverhältnis (Oxyimid:Flammschutzmittel) von 99:1 bis 1:99, bevorzugt von 5 : 95 bis 50 : 50, besonders bevorzugt von 10 : 90 bis 30 : 70 verwendet werden.

Vorteilhaft ist ferner, wenn die organischen Oxyimide, bezogen auf die Kunststoffe, zu 0,01 bis 30 Gew.-%, bevorzugt zu 0,1 bis 20 Gew.-%, besonders bevorzugt zu 1 bis 10 Gew.-% eingesetzt werden.

Zudem betrifft die vorliegende Erfindung eine Flammgeschützte Kunststoffzusammensetzung, enthaltend oder bestehend aus
a) 50 bis 98 Gew.-Teile bevorzugt 70 bis 95 Gew.-Teile mindestens eines Kunststoffs, insbesondere mindestens eines thermoplastischen Polymers,
b) 1 bis 25 Gew.-Teile bevorzugt 2,5 bis 15 Gew.-Teile mindestens eines organischen Oxyimids, enthaltend mindestens ein Strukturelement der nachfolgend abgebildeten Formel I 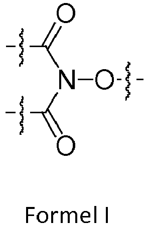 wobei das organische Oxyimid Halogen-frei ist,
c) 1 bis 25 Gew.-Teile bevorzugt 2,5 bis 15 Gew.-Teile mindestens eines weiteren Flammschutzmittels.

Gemäß einer bevorzugten Ausführungsform kann die flammgeschützte Kunststoffzusammensetzung zusätzlich
a) bis 40 Gew.-Teile mindestens eines Verstärkungs- oder Füllstoffes und/oder
b) bis 5 Gew.-Teile mindestens eines Additivs aus der Klasse der phenolischen Antioxidantien, Phosphite, Säurefänger, gehinderten Amine, Dispergiermittel sowie Kombinationen hiervon
enthalten.

Zusätzlich können Zusatzstoffe ausgewählt aus der Gruppe bestehend aus UV-Absorber, der Lichtstabilisatoren, der Stabilisatoren, der Hydroxylamine, der Benzofuranone, der Metalldesaktivatoren, der Füllstoffdesaktivatoren, der Nukleierungsmittel, Schlagzähigkeitsverbesserer, Weichmacher, Gleitmittel, Rheologiemodifikatoren, Verarbeitungshilfsmittel, Pigmente, Farbstoffe, optische Aufheller, antimikrobielle Wirkstoffe, Antistatika, Slipmittel, Antiblockmittel, Kopplungsmittel, Dispergiermittel, Kompatibilisatoren, Sauerstofffänger, Säurefänger, Markierungsmittel oder Antifoggingmittel eingesetzt werden. In bevorzugter Ausführungsform enthalten die Zusammensetzungen insbesondere Säurefänger, z.B. auf Basis von Salzen langkettiger Säuren wie z.B. Calciumstearat, Magnesiumstearat, Zinkstearat, Calciumlactat oder von Hydrotalciten und/oder Stabilisatoren aus der Gruppe der phenolischen Antioxidantien und der Phosphite/Phosphonite und/oder Lichtstabilisatoren aus der Gruppe der gehinderten Amine (HALS) und/oder Dispergiermittel .

Geeignete Lichtstabilisatoren sind beispielsweise Verbindungen auf der Basis von 2-(2'-Hydroxyphenyl)benzotriazolen, 2-Hydroxybenzophenonen, Estern von Benzoesäuren, Acrylaten, Oxamiden und 2-(2-Hydroxyphenyl)-1,3,5-Triazinen.

Geeignete 2-(2'-Hydroxyphenyl)benzotriazole sind beispielsweise 2-(2'-Hydroxy-5'methylphenyl)benzotriazol, 2-(3',5'-Di-*tert*-butyl-2'-hydroxyphenyl)benzotriazol, 2-(5'-*tert*-Butyl-2'-hydroxy-phenyl)benzotriazol, 2-(2'-Hydroxy-5'-(1,1,3,3-tetramethylbutyl)phenyl)benzotriazol, 2-(3',5'-Di-*tert*-butyl-2'-hydroxyphenyl)-5-chlorobenzotriazol, 2-(3'-*tert*-Butyl-2'-hydroxy-5'-methylphenyl-5-chlorobenzotriazol, 2-(3'-*sec*-Butyl-5'-*tert*-butyl-2'-hydroxyphenyl)benzotriazol, 2-(2'-Hydroxy-4'-octyloxyphenyl)benzotriazol, 2-(3',5'-Di-*tert*-amyl-2'-hydroxyphenyl)benzotriazol, 2-(3',5'-Bis(α,α-dimethylbenzyl)-2'-hydroxyphenyl)benzotriazol, 2-(3'-*tert*-Butyl-2'-hydroxy-5'-(2-octyloxycarbonylethyl)phenyl)-5-chlorobenzotriazol, 2-(3'-*tert*-Butyl-5'-[2-(2-ethylhexyloxy)carbonylethyl]-2'-hydroxyphenyl)-5-chlorobenzotriazol, 2-(3'-*tert*-Butyl-2'-hydroxy-5'-(2-methoxycarbonylethyl)phenyl)-5-chlorobenzotriazol, 2-(3'-*tert*-Butyl-2'-hydroxy-5'-(2-methoxycarbonylethyl)phenyl)benzotriazol, 2-(3'-*tert*-Butyl-2'-hydroxy-5'-(2-octyloxycarbonylethyl)phenyl)benzotriazol, 2-(3'-*tert*-Butyl-5'-[2-(2-ethylhexyloxy)carbonylethyl]-2'-hydroxyphenyl)benzotriazol, 2-(3'-Dodecyl-2'-hydroxy-5'-methylphenyl)benzotriazol, 2-(3'-*tert*-Butyl-2'-hydroxy-5'-(2-isooctyloxycarbonylethyl)phenylbenzotriazol, 2,2'-Methylenbis[4-(1,1,3,3-tetramethylbutyl)-6-benzotriazol-2-ylphenol]; das Produkt der Umesterung von 2-[3'-*tert*-Butyl-5'-(2-methoxycarbonylethyl)-2'-hydroxyphenyl]-2H-benzotriazol mit Polyethylenglycol 300; [R-CH₂CH₂-COO-CH₂CH₂-]-₂, wobei R = 3'-*tert*-Butyl-4'-hydroxy-5'-2H-benzotriazol-2-ylphenyl, 2-[2'-Hydroxy-3'-(α,α-dimethylbenzyl)-5'-(1,1,3,3-tetramethylbutyl)phenyl]benzotriazol, 2-[2'-hydroxy-3'-(1,1,3,3-tetramethylbutyl)-5'-(α,α-dimethylbenzyl)phenyl]benzotriazol

Geeignete 2-Hydroxybenzophenone sind beispielsweise 4-Hydroxy-, 4-Methoxy-, 4-Octyloxy-, 4-Decyloxy- 4-Dodecyloxy, 4-Benzyloxy, 4,2',4'-Trihydroxy- und 2'-Hydroxy-4,4'-dimethyoxy-Derivate der 2-Hydroxybenzophenone.

Geeignete Acrylate sind beispielsweise Ethyl-α-cyano-β,β-diphenylacrylat, Isooctyl-α-cyano-β,β-diphenylacrylat, Methyl-α-carbomethoxycinnamat, Methyl-α-cyano-β-methyl-p-methoxycinnamat, Butyl-α-cyano-β-methyl-p-methoxycinnamat, Methyl-α-carbomethoxy-p-methoxycinnamat und N-(β-carbomethoxy-β-cyanovinyl)-2-methylindolin.

Geeignete Ester von Benzoesäuren sind beispielsweise 4-*tert-*Butylphenylsalicylat, Phenylsalicylat, Octylphenylsalicylat, Dibenzoylresorcinol, Bis(4-*tert*-butylbenzoyl)resorcinol, Benzoylresorcinol, 2,4-Di-*tert*-butylphenyl-3,5-di-*tert*-butyl-4-hydroxybenzoat, Hexadecyl-3,5-di-*tert*-butyl-4-hydroxybenzoat, Octadecyl-3,5-di-*tert*-butyl-4-hydroxybenzoat, 2-Methyl-4,6-di-*tert*-butylphenyl-3,5-di-*tert*-butyl-4-hydroxybenzoat.

Geeignete Oxamide sind beispielsweise 4,4'-Dioctyloxyoxanilid, 2,2'-diethoxyoxanilid, 2,2'-Dioctyloxy-5,5'-di-*tert*-butoxanilid, 2,2'-didodecyloxy-5,5'-di-*tert*-butoxanilid, 2-Ethoxy-2'-ethyloxanilid, N,N'-Bis(3-dimethylaminopropyl)oxamid, 2-Ethoxy-5-*tert*-butyl-2'-ethoxanilid und seine Mischungen mit 2-Ethoxy-2'-ethyl-5,4'-di-*tert*-butoxanilid, Mischungen von o- und p-Methoxy-disubstituierten Oxaniliden und Mischungen von o- und p-Ethoxy-disubstituierten Oxaniliden.

Geeignete 2-(2-Hydroxyphenyl)-1,3,5-Triazine sind beispielsweise 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxypropoxy)-phenyl]-4,6-bis(2,4-dimethyl)-1,3,5-triazin, 2-[2-Hydroxy-4-(2-hydroxy-3-octyloxypropyloxy)phenyl]-4,6-bis(2,4-dimethyl)-1,3,5-triazin, 2-[4-(Dodecyloxy/Tridecyloxy-2-hydroxypropoxy)-2-hydroxyphenyl]-4,6-bis(2,4-dimethylphenyl)-1,3,5-triazin, 2-[2-Hydroxy-4-(2-hydroxy-3-dodecyloxypropoxy)phenyl]-4,6-bis(2,4-dimethylphenyl-1,3,5-triazin, 2-(2-Hydroxy-4-hexyloxy)phenyl-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxypropoxy)phenyl]-1,3,5-triazin, 2-(2-Hydroxyphenyl)-4-(4-methoxyphenyl)-6-phenyl-1,3,5-triazin, 2-{2-Hydroxy-4-[3-(2-ethylhexyl-1-oxy)-2-hydroxypropyloxy]phenyl}-4,6-bis(2,4-dimethylphenyl-1,3,5-triazin.

Geeignete Metalldesaktivatoren sind beispielsweise N,N'-Diphenyloxamid, N-Salicylal-N'-salicyloylhydrazin, N,N'-Bis(salicyloyl)hydrazin, N,N'-Bis(3,5-di-tert-butyl-4-hydroxyphenylpropionyl)hydrazin, 3-Salicyloylamino-1,2,4-triazol, Bis(benzyliden)oxalyldihydrazid, Oxanilid, Isophthaloyldihydrazid, Sebacoylbisphenylhydrazid, N,N'-Diacetyladipoyldihydrazid, N,N'-Bis(salicyloyl)oxylyldihydrazid, N,N'-Bis(salicyloyl)thiopropionyldihydrazid.

Insbesondere als Metalldesaktivatoren geeignet sind die folgenden Strukturen:

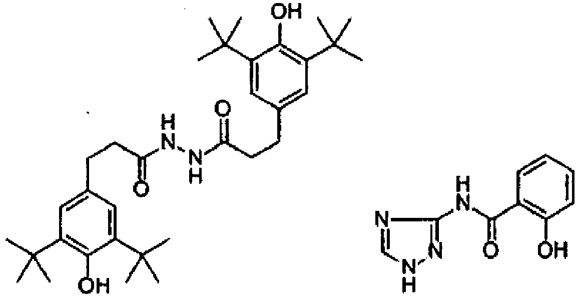

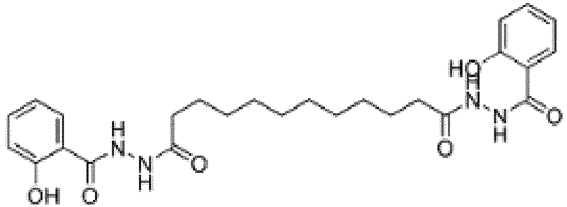

Geeignete phenolische Antioxidantien sind beispielsweise:
Alkylierte Monophenole, wie z.B. 2,6-Di-*tert*-butyl-4-methylphenol, 2-*tert-*Butyl-4,6-dimethylphenol, 2,6-Di-*tert*-butyl-4-ethylphenol, 2,6-Di-tert-butyl-4-n-butylphenol, 2,6-Di-*tert*-butyl-4-isobutylphenol, 2,6-Dicyclopentyl-4-methylphenol, 2-(α-Methylcyclohexyl)-4,6-dimethylphenol, 2,6-Dioctadecyl-4-methylphenol, 2,4,6-Tricyclohexylphenol, 2,6-Di-*tert*-butyl-4-methoxymethylphenol, lineare oder verzweigte Nonylphenole, wie z.B. 2,6-Dinonyl-4-methylphenol, 2,4-Dimethyl-6-(1'-methylundec-1'-yl)phenol, 2,4-Dimethyl-6-(1'-methylheptadec-1'-yl)phenol, 2,4-Dimethyl-6-(1'-methyltridec-1'-yl)phenol und Mischungen hiervon;
Alkylthiomethylphenole, wie z.B. 2,4-Dioctylthiomethyl-6-*tert*-butylphenol, 2,4-Dioctylthiomethyl-6-methylphenol, 2,4-Dioctylthiomethyl-6-ethylphenol, 2,6-Didodecylthiomethyl-4-nonylphenol;
Hydrochinone und alkylierte Hydrochinone, wie z.B. 2,6-Di-*tert*-butyl-4-methyoxyphenol, 2,5-Di-*tert*-butylhydrochinon, 2,5-Di-*tert*-amylhydrochinon, 2,6-Diphenyl-4-octadecyloxyphenol, 2,6-Di-*tert*-butylhydrochinon, 2,5-Di-*tert-*butyl-4-hydroxyanisol, 3,5-Di-*tert*-butyl-4-hydroxyanisol, 3,5-Di-*tert*-butyl-4-hydroxyphenylstearat, Bis(3,5-di-*tert*-butyl-4-hydroxylphenyl)adipat;
Tocopherole, wie z.B. α-, β-, γ-, δ-Tocopherol und Mischungen aus diesen (Vitamin E);
Hydroxylierte Thiodiphenylether, wie z.B. 2,2'-Thiobis(6-*tert*-butyl-4-methylphenol), 2,2'-Thiobis(4-octylphenol), 4,4'-Thiobis(6-*tert*-butyl-3-methylphenol), 4,4'-Thiobis(6-*tert*-butyl-2-methylphenol), 4,4'-Thiobis(3,6-di-*sec*-amylphenol), 4,4'-Bis(2,6-dimethyl-4-hydroxyphenyl)disulfid;
Alkylidenbisphenole, wie z.B. 2,2'Methylenbis(6-*tert*-butyl-4-methylphenol), 2,2'-Methylenbis(6-*tert*-butyl-4-ethylphenol), 2,2'-Methylenbis[4-methyl-6-(α-methylcyclohexyl)phenol], 2,2'-Methylenbis(4-methyl-6-cyclhexylphenol), 2,2'-Methylenbis(6-nonyl-4-methylphenol), 2,2'-Methylenbis(4,6-di-*tert-*butylphenol), 2,2'-Ethylidenbis(4,6-di-*tert*-butylphenol), 2,2'-Ethylidenbis(6-*tert*-butyl-4-isobutylphenol), 2,2'-Methylenbis[6-(α-methylbenzyl)-4-nonylphenol], 2,2'-Methylenbis[6-(α,α-dimethylbenzyl)-4-nonylphenol], 4,4'-Methylenbis(2,6-di-*tert*-butylphenol,4,4'-Methylenbis(6-*tert*-butyl-2-methylphenol), 1,1-bis(5-*tert*-butyl-4-hydroxy-2-methylphenyl)butan, 2,6-Bis(3-*tert*-butyl-5-methyl-2-hydroxybenzyl)-4-methylphenol, 1,1,3-Tris(5-*tert-*butyl-4-hydroxy-2-methylphenyl)butan, 1,1-bis(5-*tert*-butyl-4-hydroxy-2-methylphenyl)-3-n-dodecylmercaptobutan, Ethylenglycol-bis[3,3-bis(3'-*tert-*butyl-4'-hydroxyphenyl)butyrat], Bis(3-*tert*-butyl-4-hydroxy-5-methylphenyl)dicyclopentadien, Bis[2-(3'-*tert*-butyl-2'-hydroxy-5'-methylbenzyl)-6-*tert*-butyl-4-methylphenyl]terephthalat, 1,1-Bis-(3,5-dimethyl-2-hydroxyphenyl)butan, 2,2-Bis(3,5-di-*tert*-butyl-4-hydroxyphenyl)propan, 2,2-Bis-(5-*tert*-butyl-4-hydroxy-2-methylphenyl)-4-n-dodecylmercaptobutan, 1,1,5,5-Tetra(5-*tert*-butyl-4-hydroxy-2-methylphenyl)pentan;
O-, N- und S-Benzyl-Verbindungen, wie z.B. 3,5,3',5'-Tetra-*tert*-butyl-4,4'-dihydroxydibenzylether, Octadecyl-4-hydroxy-3,5-dimethylbenzylmercaptoacetat, Tridecyl-4-hydroxy-3,5-di-*tert-*butylbenzylmercaptoacetat, Tris(3,5-di-*tert*-butyl-4-hydroxybenzyl)amin, , Bis(4-*tert*-butyl-3-hydroxy-2,6-dimethylbenzyl)dithioterephthalat, Bis(3,5-di-*tert*-butyl-4-hydroxybenzyl)sulfid, Isooctyl-3,5-di-*tert*-butyl-4-hydroxybenzylmercaptoacetat;
Hydroxybenzylierte Malonate, wie z.B. Dioctadecyl-2,2-bis(3,5-di-*tert*-butyl-2-hydroxybenzyl)malonat, Dioctadecyl-2-(3-*tert*-butyl-4-hydroxy-5-methylbenzyl)malonat, Didodecylmercaptoethyl-2,2-bis(3,5-di-*tert*-butyl-4-hydroxybenzyl)malonat, Bis[4-(1,1,3,3-tetramethylbutyl)phenyl]-2,2-bis(3,5-di-*tert*-butyl-4-hydroxybenzyl)malonat;
Aromatische Hydroxybenzylverbindungen, wie z.B. 1,3,5-Tris(3,5-di-*tert*-butyl-4-hydroxybenzyl)-2,4,6-trimethylbenzol, 1,4-Bis(3,5-di-*tert*-butyl-4-hydroxybenzyl)-2,3,5,6-tetramethylbenzol, 2,4,6-Tris(3,5-di-*tert*-butyl-4-hydroxybenzyl)phenol;
Triazinverbindungen, wie z.B. 2,4-Bis(octylmercapto)-6-(3,5-di-*tert*-butyl-4-hydroxyanilino)-1,3,5-triazin, 2-Octylmercapto-4,6-bis(3,5-di-*tert*-butyl-4-hydroxyanilino)-1,3,5-triazin, 2-Octylmercapto-4,6-bis(3,5-di-*tert*-butyl-4-hydroxyphenoxy)-1,3,5-triazin, 2,4,6-Tris(3,5-di-*tert*-butyl-4-hydroxyphenoxy)-1,2,3-triazin, 1,3,5-Tris(3,5-di-*tert*-butyl-4-hydroxybenzyl)isocyanurat, 1,3,5-Tris(4-*tert*-butyl-3-hydroxy-2,6-dimethylbenzyl)isocyanurat, 2,4,6-Tris(3,5-di-tert-butyl-4-hydroxphenylethyl)-1,3,5-triazin, 1,3,5-Tris(3,5-di-*tert*-butyl-4-hydroyphenylpropionyl)hexahydro-1,3,5-triazin, 1,3,5-Tris(3,5-dicyclohexyl-4-hydroxybenzyl)isocyanurat;
Benzylphosphonate, wie z.B. Dimethyl-2,5-di-*tert*-butyl-4-hydroxybenzylphosphonat, Dietyhl-3,5-di-*tert*-butyl-4-hydroxybenzylphosphonat, Dioctadecyl-3,5-di-*tert*-butyl-4-hydroxybenzylphosphonat, Dioctadecyl-5-*tert*-butyl-4-hydroxy-3-methylbenzylphosphonat, das Calciumsalz des Monoethylesters der 3,5-Di-*tert*-butyl-4-hydroxybenzylphosphonsäure;
Acylaminophenole, wie z.B. 4-Hydroxylauranilid, 4-Hydroxystearanilid, Octyl-N-(3,5-di-*tert*-butyl-4-hydroxyphenyl)carbamat;
Ester der β-(3,5-Di-*tert*-butyl-4-hydroxyphenyl)propionsäure mit ein- oder mehrwertigen Alkoholen, z.B. Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythritol, Tris(hydroxyethyl)isocyanurat, N,N'-Bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan.
Ester der β-(5-*tert*-Butyl-4-hydroxy-3-methylphenyl)propionsäure mit ein- oder mehrwertigen Alkoholen, z.B. Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythritol, Tris(hydroxyethyl)isocyanurat, N,N'-bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan, 3,9-Bis[2-{3-(3-*tert*-butyl-4-hydroxy-5-methylphenyl)propionyloxy}-1,1-dimethylethyl]-2,4,8,10-tetraoxaspiro[5.5]undecan;
Ester der β-(3,5-Dicyclohexyl-4-hydroxyphenyl)propionsäure mit ein- oder mehrwertigen Alkoholen, z.B. Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythritol, Tris(hydroxyethyl)isocyanurat, N,N'-bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan;
Ester der 3,5-Di-*tert*-butyl-4-hydroxyphenyl)essigsäure mit ein- oder mehrwertigen Alkoholen, z.B. Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythritol, Tris(hydroxyethyl)isocyanurat, N,N'-bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan;
Amide der β-(3,5-Di-*tert*-butyl-4-hydroxyphenyl)propionsäure, wie z.B. N,N'-Bis(3,5-di-*tert*-butyl-4-hydroxyphenylpropionyl)hexamethylendiamid, N,N'-Bis(3,5-di-*tert*-butyl-4-hydroxyphenylpropionyl)hexamethylendiamid, N,N'-Bis(3,5-di-*tert*-butyl-4-hydroxyphenylpropionyl)hexamethylendiamid, N,N'-Bis(3,5-di-*tert*-butyl-4-hydroxyphenylpropionyl)hydrazid, N,N'-Bis[2-(3-[3,5-di-*tert*-butyl-4-hydroxyphenyl]propionyloxy)ethyl]oxamid (Naugard®XL-1, vertrieben durch Uniroyal);

### Ascorbinsäure (Vitamin C).

Besonders bevorzugte phenolische Antioxidantien sind:
Octadecyl-3-(3,5-di-*tert*-butyl-4-hydroxyphenyl)propionat, Pentaerythritol-tetrakis[3-(3,5-di-*tert*-butyl-4-hydroxyphenyl)propionat, Tris(3,5-di-*tert*-butyl-4-hydroxyphenyl)isocyanurat, 1,3,5-Trimethyl-2,4,6-tris(3,5-di-*tert*-butyl-4-hydroxyphenyl)isocyanurat, 1,3,5-Trimethyl-2,4,6-tris(3,5-di-tert-butyl-4-hydroxybenzyl)benzol, Triethylenglycol-bis[3-(3-*tert*-butyl-4-hydroxy-5-methylphenyl)propionat, N,N'-Hexan-1,6-diyl-bis[3-(3,5-di-*tert*-butyl-4-hydroxyphenyl)propionsäureamid.

Geeignete Phosphite/Phosphonite sind beispielsweise:
Triphenylphosphit, Diphenylalkylphosphite, Phenyldialkylphosphite, Tri(nonylphenyl)phosphit, Trilaurylphosphite, Trioctadecylphosphit, Distearylpentaerythritoldiphosphit, Tris-(2,4-di-*tert*-butylphenyl)phosphit, Diisodecylpentaerythritoldiphosphit, Bis(2,4-di-*tert-*butylphenyl)pentaerythritoldiphosphit, Bis(2,4-di-cumylphenyl)pentaerythritoldiphosphit, Bis(2,6-di-*tert*-butyl-4-methylphenyl)pentaerythritoldiphosphit, Diisodecyloxypentaerythritoldiphosphit, Bis(2,4-di-*tert*-butyl-6-methylphenyl)pentaerythritoldiphosphit, Bis(2,4,6-tris(*tert-*butylphenyl)pentaerythritoldiphosphit, Tristearylsorbitoltriphosphit, Tetrakis(2,4-di-*tert*-butylphenyl)-4,4'-biphenylendiphosphonit, 6-Isooctyloxy-2,4,8,10-tetra-*tert*-butyl-12H-dibenz[d,g]-1,3,2-dioxaphosphocin, Bis(2,4-di-*tert*-butyl-6-methylphenyl)methylphosphit, Bis(2,4-di-*tert*-butyl-6-methylphenyl)ethylphosphit, 6-Fluoro-2,4,8,10-tetra-*tert*-butyl-12-methyl-dibenz[d,g]-1,3,2-dioxaphosphocin, 2,2'2"-Nitrilo[triethyltris(3,3",5,5'-tetra-*tert*-butyl-1,1'-biphenyl-2,2'-diyl)phosphit], 2-Ethylhexyl(3,3',5,5'-tetra-*tert-*butyl-1,1'-biphenyl-2,2'-diyl))phosphit, 5-Butyl-5-ethyl-2-(2,4,6-tri-*tert-*butylphenoxy)-1,3,2-dioxaphosphiran.

Besonders bevorzugte Phosphite/Phosphonite sind:

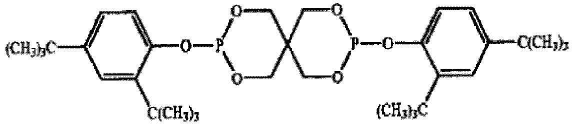

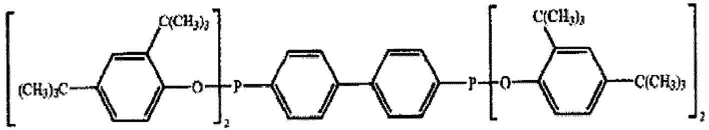

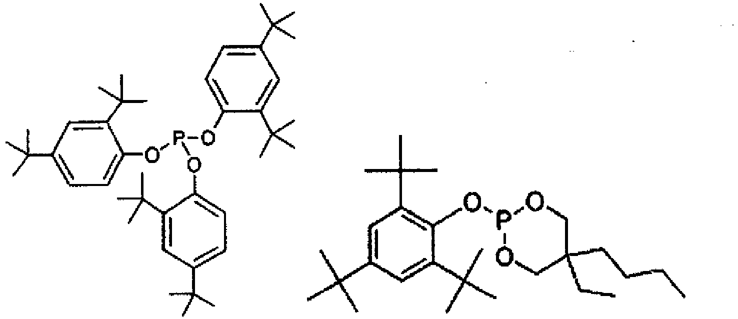

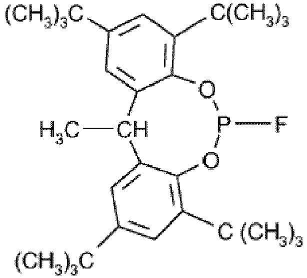

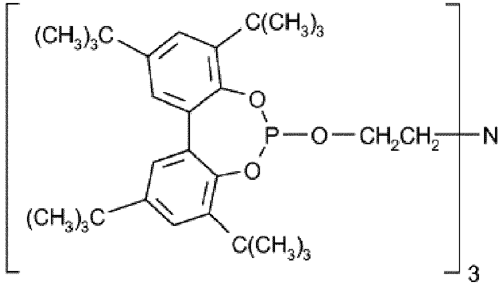

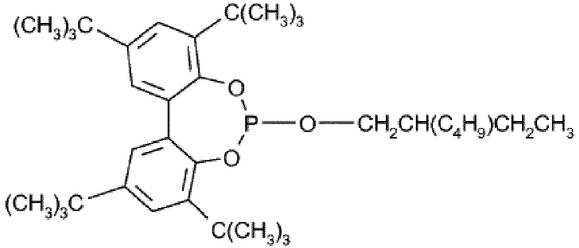

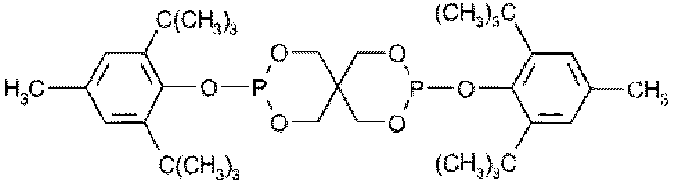

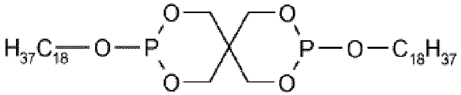

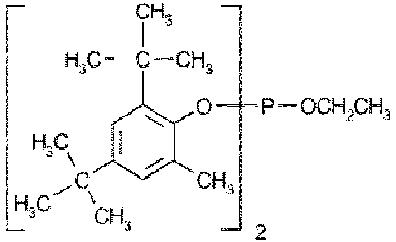

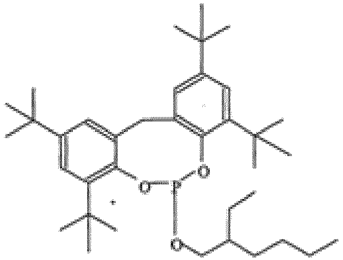

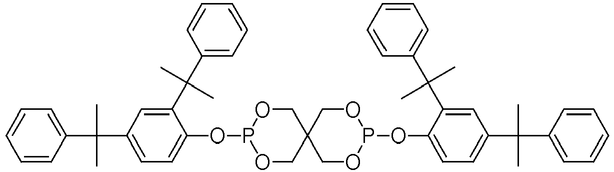

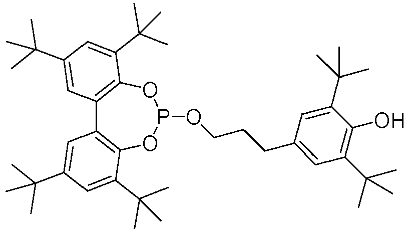

Weitere geeignete Stabilisatoren sind aminische Antioxidantien. Geeignete aminische Antioxidantien sind beispielsweise:
N,N'-Di-isopropyl-p-phenylendiamin, N,N'-Di-*sec*-butyl-p-phenylendiamin, N,N'-Bis(1,4-dimethylpentyl)-p-phenylendiamin, N,N'-Bis(1-ethyl-3-methylpentyl)-p-phenylendiamin, N,N'-Bis(1-methylheptyl)-p-phenylendiamin, N,N'-Dicyclohexyl-p-phenylendiamin, N,N'-Diphenyl-p-phenylendiamin, N,N'-Bis(2-naphthyl)-p-phenylendiamin, N-Isopropyl-N'-phenyl-p-phenylendiamin, N-(1,3-Dimethylbutyl)-N'-phenyl-p-phenylendiamin, N-(1-Methylheptyl)-N'-phenyl-p-phenylendiamin, N-Cyclohexyl-N'-phenyl-p-phenylendiamin, 4-(p-Toluolsulfamoyl)diphenylamin, N,N'-Dimethyl-N,N'-di-sec-butyl-p-phenylendiamin, Diphenylamin, N-Allyldiphenylamin, 4-Isopropoxydiphenylamin, N-Phenyl-1-naphthylamin, N-(4-tert-Octylphenyl)-1-naphthylamin, N-Phenyl-2-naphthylamin, octyliertes Diphenylamin, z.B. p,p'-Di-tert-octyldiphenylamin, 4-n-Butylaminophenol, 4-Butyrylaminophenol, 4-Nonanoylaminophenol, 4-Dodecanoylaminophenol, 4-Octadecanoylamino-phenol, Bis(4-methoxyphenyl)amin, 2,6-Di-tert-butyl-4-dimethylaminomethyl-phenol, 2,4'-Diaminodiphenylmethan, 4,4'-Diaminodiphenylmethan, N,N,N',N'-Tetra-methyl-4,4'-diaminodiphenylmethan, 1,2-Bis[(2-methyl-phenyl)amino]ethan, 1,2-Bis(phenylamino)propan, (o-Tolyl)biguanid, Bis[4-(1',3'-dimethylbutyl)phenyl]amin, tert-octyliertes N-Phenyl-1-naphthylamin, ein Gemisch aus mono- und dialkylierten *tert*-Butyl/*tert*-Octyldiphenylaminen, ein Gemisch aus mono- und dialkylierten Nonyldiphenylaminen, ein Gemisch aus mono- und dialkylierten Dodecyldiphenylaminen, ein Gemisch aus mono- und dialkylierten Isopropyl/Isohexyl-diphenylaminen, ein Gemisch aus mono- und dialkylierten *tert*-Butyldiphenylaminen, 2,3-Dihydro-3,3-dimethyl-4H-1,4-benzothiazin, Phenothiazin, ein Gemisch aus mono- und dialkylierten *tert*-Butyl/*tert*-Octylphenothiazinen, ein Gemisch aus mono- und dialkylierten *tert*-Octylphenothiazinen, N-Allylphenothiazin, N,N,N',N'-Tetraphenyl-1,4-diaminobut-2-en sowie Mischungen oder Kombinationen hiervon.

Weitere geeignete aminische Antioxidantien sind Hydroxylamine bzw. N-oxide (Nitrone), wie z.B. N,N-Dialkylhydroxylamine, N,N-Dibenzylhydroxylamin, N,N-Dilaurylhydroxylamin, N,N-Distearylhydroxylamin, N-Benzyl-α-phenylnitron, N-Octadecyl-α-hexadecylnitron, sowie Genox EP (Chemtura) gemäß der Formel:

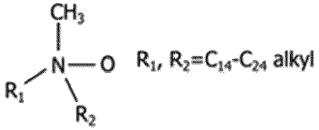

Weitere geeignete Stabilisatoren sind Thiosynergisten. Geeignete Thiosynergisten sind beispielsweise Distearylthiodipropionat, Dilauryldipropionat oder die Verbindung gemäß der folgenden Formel:

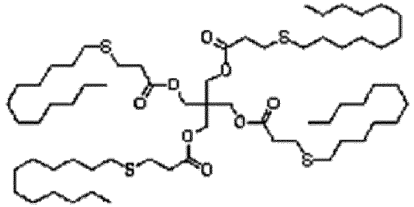

Weitere geeignete Stabilisatoren insbesondere für Polyamide sind Kupfersalze wie z.B. Kupfer-(I)-iodid, Kupfer-(I)-bromid oder Kupferkomplexe wie z.B. Triphenylphosphin-Kupfer-(I)-Komplexe.

Geeignete gehinderte Amine sind beispielsweise 1,1-Bis(2,2,6,6-tetramethyl-4-piperidyl)succinat, Bis(1,2,2,6,6-pentamethyl-4-piperidyl)sebazat, Bis(1-octyloxy-2,2,6,6-tetramethyl-4-piperidyl)sebazat, Bis(1,2,2,6,6-pentamethyl-4-piperidyl)-n-butyl-3,5-di-tert-butyl-4-hydroxybenzylmalonat, das Kondensationsprodukt aus 1-(2-Hydroxyethyl)-2,2,6,6-tetramethyl-4-hydroxypiperidin und Succinsäure, lineare oder zyklische Kondensationsprodukte von N,N'-Bis(2,2,6,6-tetramethyl-4-piperidyl)hexamethylendiamin und 4-*tert*-Octylamino-2,6-di-chloro-1,3,5-triazin, Tris(2,2,6,6-tetramethyl-4-piperidyl)nitrilotriacetat, Tetrakis(2,2,6,6-tetra-methyl-4-piperidyl)-1,2,3,4-butantetracarboxylat, 1,1'-(1,2-Ethandiyl)-bis(3,3,5,5-tetramethylpiperazinon), 4-Benzoyl-2,2,6,6-tetramethylpiperidin, 4-Stearyloxy-2,2,6,6-tetramethylpiperidin, lineare oder zyklische Kondensationsprodukte aus N,N'-Bis(2,2,6,6-tetramethyl-4-piperidyl)hexamethylendiamin und 4-Morpholino-2,6-dichloro-1,3,5-triazin das Reaktionsprodukt von 7,7,9,9-Tetramethyl-2-cycloundecyl-1-oxa-3,8-diaza-4-oxospiro-[4,5]decan und Epichlorhydrin.

Geeignete Dispergiermittel sind beispielsweise:
Polyacrylate, z.B. Copolymere mit langkettigen Seitengruppen, Polyacrylat-Blockcopolymere, Alkylamide: z.B. N,N'-1,2-Ethandiylbisoctadecanamid Sorbitanester, z.B. Monostearylsorbitanester, Titanate und Zirconate, reaktive Copolymere mit funktionellen Gruppen z.B. Polypropylen-co-Acrylsäure, Polypropylen-co-Maleinsäureanhydrid, Polyethylen-co-Glycidylmethacrylat, Polystyrol-alt-Maleinsäureanhydrid-Polysiloxane: z.B. Dimethylsilandiol-Ethylenoxid Copolymer, Polyphenylsiloxan Copolymer, Amphiphile Copolymere: z.B. Polyethylen-block-Polyethylenoxid, Dendrimere, z.B. hydroxylgruppenhaltige Dendrimere.

Geeignete Nukleierungsmittel sind beispielsweise Talkum, Alkali oder Erdalkalisalze von mono- und polyfunktionellen Carbonsäuren wie z.B. Benzoesäure, Bernsteinsäure, Adipinsäure, z.B. Natriumbenzoat, Zinkglycerolat, Aluminium-hydroxy-bis(4-*tert*-butyl)benzoat, Benzylidensorbitole wie z.B. 1,3:2,4-Bis(Benzyliden)sorbitol oder 1,3:2,4-Bis(4-Methylbenzyliden)sorbitol, 2,2'-Methylen-bis-(4,6-di-*tert*-butylphenyl)phosphat, sowie Trisamide wie z.B. gemäß der folgenden Strukturen

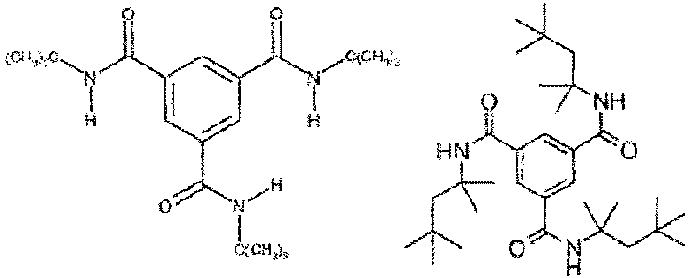

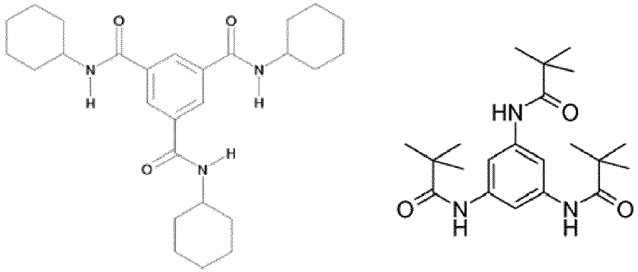

Geeignete Füllstoffe und Verstärkungsstoffe sind beispielsweise synthetische oder natürliche Materialien wie z.B. Calciumcarbonat, Silikate, Glasfasern, Glaskugeln (massiv oder hohl), Talkum, Glimmer, Kaolin, Bariumsulfat, Metalloxide und Metallhydroxide, Ruß, Graphit, Kohlenstoffnanoröhrchen, Graphen, Holzmehl oder Fasern von Naturprodukten wie z.B. Cellulose oder synthetische Fasern. Weitere geeignete Füllstoffe sind Hydrotalcite oder Zeolithe oder Schichtsilikate wie z.B. Montmorillonit, Bentonit, Beidelit, Mica, Hectorit, Saponit, Vermiculit, Ledikit, Magadit, Illit, Kaolinit, Wollastonit, Attapulgit.

Geeignete Pigmente können anorganischer oder organischer Natur sein. Geeignete anorganische Pigmente sind beispielsweise Titandioxid, Zinkoxid, Zinksulfid, Eisenoxid, Ultramarin, Ruß. Geeignete organische Pigmente sind beispielsweise Anthrachinone, Anthanthrone, Benzimidazolone, Chinacridone, Diketopyrrolopyrrole, Dioxazine, Indanthrone, Isoindolinone, Azo-Verbindungen, Perylene, Phthalocyanine oder Pyranthrone. Weitere geeignete Pigmente sind Effektpigmente auf Metallbasis oder Perlglanzpigmente auf Metalloxid-Basis.

Optische Aufheller sind beispielsweise Bisbenzoxazole, Phenylcumarine oder Bis(styryl)biphenyle und insbesondere optische Aufheller der Formeln:

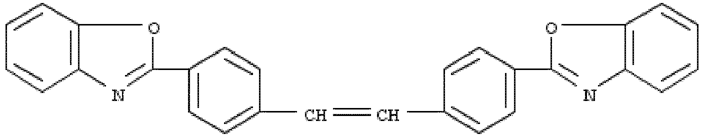

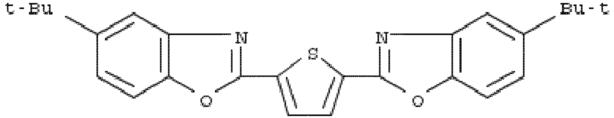

Geeignete Füllstoffdesaktivatoren sind beispielsweise Epoxide wie z.B. Bisphenol-A-diglycidylether, Polysiloxane, Polyacrylate insbesondere Blockcopolymere wie Polymethacrylsäure-polyalkylenoxid.

Geeignete Antistatika sind beispielsweise ethoxylierte Alkylamine, Fettsäureester, Alkylsulfonate und Polymere wie z.B. Polyetheramide.

Weiterhin betrifft die vorliegende Erfindung eine Formmasse, ein Formteil, einen Lack oder eine Beschichtung herstellbar aus einer zuvor beschriebenen flammgeschützten Kunststoffzusammensetzung insbesondere in Form von Spritzgussteilen, Folien oder Filmen, Beschichtungen oder Lacken, Schäumen, Fasern, Kabeln und Rohren, Profilen, Bändchen, Membranen, wie z.B. Geomembranen, Klebstoffen, die über Extrusion, Spritzguss, Blasformen, Kalandrieren, Pressverfahren, Spinnprozesse oder Streich- und Beschichtungsprozesse hergestellt werden z.B. für die Elektro- und Elektronikindustrie, Bauindustrie, Transportindustrie (Auto, Flugzeug, Schiff, Bahn), für medizinische Anwendungen, für Haushalts- und Elektrogeräte, Fahrzeugteile, Konsumartikel, Möbel, Textilien. Ein weiterer Einsatzbereich sind Lacke, Farben und Beschichtungen (Coatings).

Beispielsweise können die erfindungsgemäßen Zusammensetzungen für Marineanwendungen (Pontoons, Planken, Boote), Autoanwendungen (Stoßfänger, Batterien, Verkleidungsteile, Benzintanks, Kabel, Leitungen etc.), Flugzeugteile, Eisenbahnteile, Fahrrad- und Motorradteile, Raumfahrtanwendungen wie z.B. Satellitenteile, Gehäuseteile für Elektrogeräte wie Computer, Telefone, Drucker, Audio- und Videosysteme, Stecker, gedruckte Schaltungen, Schalter, Lampenabdeckungen, Kühlschränke, Kaffeemaschinen, Staubsauger, Rotorblätter für die Energiegewinnung, Ventilatoren, Folien für Dachkonstruktionen, Baufolien, Rohre, wie z.B. Abwasserrohre und Gasrohre, Verbindungsstücke, Drainagesysteme, Profile wie z.B. Fensterprofile oder Kabelkanäle, Wood Composites, Möbel, Fußboden, Verkleidungsplatten, künstlicher Rasen, Stadionsitze, Teppiche, Netze, Seile, Möbelteile, Matten, Gartenstühle, Flaschenkästen, Behälter und Fässer verwendet werden.

Die Erfindung betrifft ebenso ein Verfahren zur Herstellung einer zuvor beschriebenen erfindungsgemäßen Kunststoffzusammensetzung, bei dem das mindestens eine organische Oxyimid vor, nach oder gleichzeitig mit dem mindestens einen weiteren Flammschutzmittel in den mindestens einen Kunststoff bevorzugt das mindestens eine thermoplastische Polymer eingearbeitet wird.

Die Einarbeitung der oben beschriebenen Flammschutzmittel und der zusätzlichen Additive in den Kunststoff erfolgt durch übliche Verarbeitungsmethoden, wobei das Polymere aufgeschmolzen und mit den Flammschutzmitteln und Zusätzen gemischt wird, vorzugsweise durch Mischer, Kneter und Extruder. Als Verarbeitungsmaschinen bevorzugt sind Extruder wie z.B. Einschneckenextruder, Zweischneckenextruder, Planetwalzenextruder, Ringextruder, Co-Kneter, die vorzugsweise mit einer Vakuumentgasung ausgestattet sind. Die Verarbeitung kann dabei unter Luft oder ggf. unter Inertgasbedingungen erfolgen. Verschiedene Flammschutzmittel und Additive können dabei separat oder als Mischung zugegeben werden, in Form von Flüssigkeiten, Pulvern, Granulaten oder kompaktierten Produkten oder ebenfalls in Form von Masterbatchen oder Konzentraten, die beispielsweise 50-80 % der erfindungsgemäßen Zusammensetzungen enthalten.

Die Erfindung betrifft ebenso eine Flammschutzzusammensetzung bestehend aus einem organischen Oxyimid, enthaltend mindestens ein Strukturelement der nachfolgend abgebildeten Formel I, 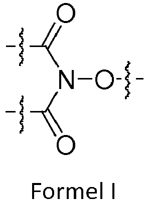 und einem weiteren Flammschutzmittel, wobei das organische Oxyimid Halogen-frei ist.

Die vorliegende Erfindung wird anhand der nachfolgenden Ausführungsbeispiele näher erläutert ohne die Erfindung auf die dort dargestellten speziellen Parameter zu beschränken.

### Synthesen:

### Verbindung 1: N-Hydroxyphthalimid, bezogen von Aldrich

### Verbindung 2: Synthese von N,N'-Dihydroxypyromellitimid

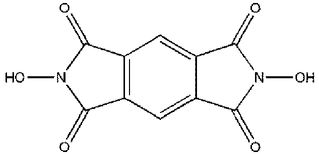

12,3 g (0,1773 mol, 2,2 Äq) Hydroxylaminhydrochlorid werden in 100 mL Pyridin gelöst. Zu der Lösung werden 17,6 g (0,0807 mol, 1 Äq) Pyromellithsäuredianhydrid zugegeben. Die Reaktionsmischung wird für 2 h bei 90 °C und anschließend für weitere 12 h bei Raumtemperatur gerührt. Der ausgefallene Rückstand wird abfiltriert und mit 5%iger Salzsäure sowie mit Wasser gewaschen. Es werden 12 g (60 %) eines weißen Produktes erhalten.
**¹H-NMR** (DMSO, 300 MHz) δ [ppm] = 8,13 (s, 2H, CH), 11,15 (s, 2H, OH).

### Verbindung 3: Synthese von N-Hydroxynaphthalimid

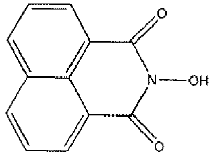

4,9 g (0,0705 mol, 1,5 Äq) Hydroxylaminhydrochlorid werden in 60 mL Pyridin gelöst. Zu der Lösung werden 9,3 g (0,0469 mol, 1 Äq) 1,8-Naphthalsäureanhydrid zugegeben. Die Reaktionsmischung wird für 20 h bei Raumtemperatur gerührt. Der ausgefallene Rückstand wird abfiltriert und mit 5%iger Salzsäure sowie mit Wasser gewaschen. Es werden 8 g (80 %) eines weißen Produktes erhalten.
**¹H-NMR** (DMSO, 300 MHz) δ [ppm] = 7,87 (t, 2H, CH), 8,49 (dd, 4H, CH), 10,73 (s, 1H, OH).

### Verbindung 4: Synthese von O,O'-Terephthaloyl-bis-N,N'-phthalimidester

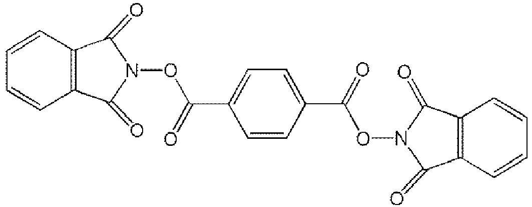

91 g *N*-Hydroxyphthalimid (0,5578 mol, 3 Äq) werden in 200 mL Pyridin unter Schutzgasatmosphäre gelöst. Zu der Lösung werden 38 g (0,1870 mol, 1 Äq) Terephthalsäuredichlorid zugegeben. Die Mischung wird für 2 h bei 60 °C und anschließend für 12 h bei Raumtemperatur gerührt. Der weiße Feststoff wird abfiltriert und mit 5%iger Salzsäure sowie mit Wasser gewaschen. Es werden 76 g (89 %) weiße Kristalle erhalten.
**¹H-NMR** (DMSO, 300 MHz) δ [ppm] = 8,05 (m, 8H, CH), 8,44 (s, 4H, CH).

### Verbindung 5: Synthese von O,O'-Succinyloyl-bis-N,N'-phthalimidester

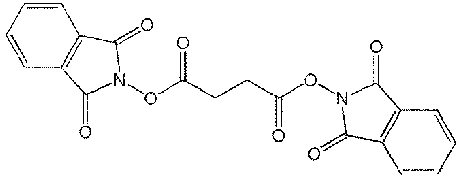

30 g (0,1840 mol, 3 Äq) *N-*Hydroxyphthalimid werden in 180 mL trockenem Pyridin unter Schutzgasatmosphäre gelöst. Zu der Lösung werden 6,75 mL (0,0613 mol, 1 Äq) Bernsteinsäuredichlorid zugetropft. Die Reaktionsmischung wird für 24 h bei Raumtemperatur gerührt. Der ausgefallene Feststoff wird abfiltriert und mit 5%iger Salzsäure sowie mit Wasser gewaschen. Die Aufreinigung erfolgt durch Umkristallisation in Dichlormethan. Es werden 15 g (60 %) weißer Kristalle erhalten.
**¹H-NMR** (DMSO, 300 MHz) δ [ppm] = 3,21 (s, 4H, CH₂), 7,96 (m, 8H, CH).

### Verbindung 6: Synthese von O,O'-Terephthaloyl-bis-N,N'-succinimidester

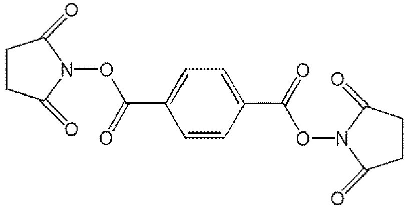

2,82 g (0,0139 mol, 1 Äq.) Terephthalsäuredichlorid werden in einem ausgeheizten Schlenkkolben unter Stickstoffstrom in 60 mL trockenem Pyridin suspendiert. Unter Schutzgasatmosphäre wird zu der Suspension tropfenweise eine zweite Lösung aus 4,79 g (0,0417 mol, 3 Äq.) *N-*Hydroxysuccinimid in 60 mL trockenem Pyridin zugegeben und die Reaktionsmischung für 24 h bei Raumtemperatur gerührt. Der ausgefallene Rückstand wird abfiltriert und mit 5%iger Salzsäure (3 x 15 mL) sowie mit Wasser gewaschen. Der erhaltene Feststoff wird im Vakuum getrocknet. Es werden 3,379 g (68 %) eines weißen Produktes erhalten.
**¹H-NMR** (DMSO, 300 MHz) δ [ppm] = 2,92 (s, 8H, CH₃), 8,34 (s, 4H, CH).

### Verbindung 7: Synthese von O,O',O"-Trimesyloyl-tri-N,N',N"-phthalimidester

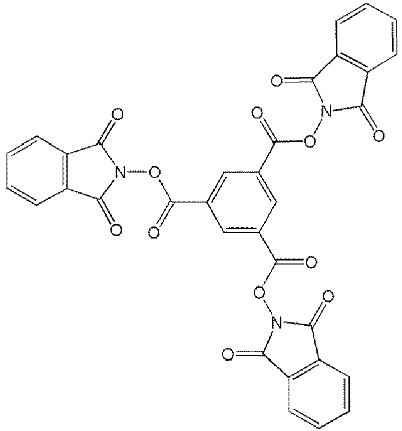

3,80 g (0,0232 mol, 3 Äq.) *N-*Hydroxyphthalimid werden mit 2,1 mL (0,026 mol, 3,4 Äq.) Pyridin in einem ausgeheizten Schlenkkolben im Stickstoffstrom in 83 mL trockenem Aceton gelöst und dann auf 5°C abgekühlt. Unter Rühren wird eine Lösung von 1,4 mL (0,0077 mol, 1 Äq.) Trimesyltrichlorid in 19 mL destilliertem Aceton zugetropft. Nach Beendigung der Zugabe wird die Mischung für 1 h bei 5°C gerührt, anschließend wird die Reaktionsmischung für 12 h bei Raumtemperatur gerührt. Der ausgefallene, weiße Feststoff wird abfiltriert und mit 5%iger Salzsäure sowie mit Wasser gewaschen. Es werden 3,9 g (78 %) eines weißen Produktes erhalten.
**¹H-NMR** (DMSO, 300 MHz) δ [ppm] = 8,04 (m, 12H, CH), 9,09 (s, 3H, CH).

### Verbindung 8: Synthese von O,O'-Terephthaloyl-bis-N,N'-naphthalimidester

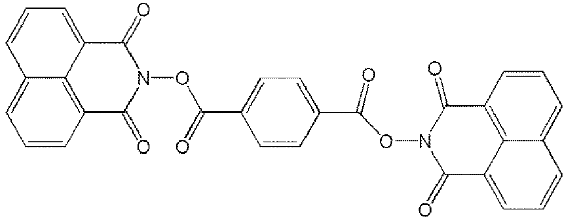

6,18 g (0,0290 mol, 2 ,5 Äq.) *N*-Hydroxynaphthalimid werden in einem ausgeheizten Schlenkkolben im Stickstoffstrom in 180 mL trockenem Pyridin bei 45 °C gelöst. Unter Schutzgasatmosphäre wird zu der Lösung 2,3 g (0,0113 mol, 1Äq.) Terephthalsäuredichlorid zugegeben. Die Reaktionsmischung wird für 1 h bei 45 °C und anschließend für weitere 20 h bei Raumtemperatur gerührt. Der ausgefallene Rückstand wird abfiltriert und mit 5%iger Salzsäure sowie mit Wasser gewaschen. Es werden 4,8 g (76 %) eines weißen Produktes erhalten.
**¹H-NMR** (ODCB, 300 MHz) δ [ppm] = 7,41 (t, 4H, CH), 7,83 (d, 4H, CH), 8,12 (s, 4H, CH), 8,34 (d, 4H, CH).

### Ausführungsbeispiele:

Die Extrusionen der Polypropylen-Proben (DOW 766-03) erfolgen bei einer Temperatur von 190 °C und einer Schneckendrehzahl von 150 U/min auf einem DSM Micro 5cc, Doppelschneckenextruder. Die Verweilzeit des Gemisches im Bypass-Betrieb beträgt 60 s. Das gewünschte Gemisch aus Polymer und Additiven (siehe nachfolgende Tabelle 1) wird zunächst in einem Becherglas manuell vermischt und in kleinen Portionen dem Mikroextruder zugeführt. Ist das Volumen des Gerätesmit Schmelze gefüllt, bleibt das Material noch 60 s (Verweilzeit) im Gerät und wird dann abgelassen.

Die erhaltenen Kunststoff-Stränge werden granuliert (Pell-Tec SD 50 pure).

Probekörper für die Brandprüfung werden aus dem Granulat bei einer Temperatur von 220°C und einem Druck von 2 t unter Verwendung einer hydraulischen 10t-Presse (Werner & Pfleiderer) hergestellt. Dazu wird das Granulat in die Pressform eingefüllt und diese in die bereits vorgeheizte Presse überführt. Bei einem Druck von 0,5 t wird das Granulat zunächst 60 s lang aufgeschmolzen. Nach Ablauf der Schmelzzeit wird der Druck auf 2 t erhöht und für weitere 3 min konstant gehalten. Unter Beibehaltung des Anpressdruckes wird die Form auf 60°C abgekühlt und danach die Probekörper entnommen. Die Probenkörper haben gemäß Norm die folgenden Dimensionen: 125 x 12,5 x 1,5 mm.

Die in der Tabelle 1 enthaltenen erfindungsgemäßen Beispiele und Vergleichsbeispiele wurden nach DIN EN 60695-11-10 geprüft und die Brennzeiten und Klassifizierung gemäß Norm erhalten:

**Tabelle 1: Zusammensetzungen in Polypropylen und Ergebnisse der Brandprüfung**

| Beispiel | Zusammensetzung Flammschutzmittel | Brennzeiten als Summe der Nachbrennzeiten von 5 Prüfkörper bei 2 Beflammungen [in Sekunden] | Klassifizierung nach DIN EN 60695-11-10 |
|---|---|---|---|
| Vergleichsbeispiel 1 (Stand der Technik) | 8% Diethylaluminiumphosphinat + 2% Disterarylhydroxylamin | 170 | Nicht klassifiziert |
| Vergleichsbeispiel 2 | 10% Diethylaluminiumphosphinat | > 200 | Nicht klassifiziert |
| Erfindungsgemäßes Beispiel 1 | 8% Diethylaluminiumphosphinat + 2% Hydroxyphthalimid | 14 | V-2 |
| Erfindungsgemäßes Beispiel 2 | 6% Diethylaluminiumphosphinat + 2% Hydroxyphthalimid | 28 | V-2 |

Diethylaluminiumphosphinat (Exolit OP 1230, Hersteller: Clariant) Distearylhydroxylamin, Hydroxyphthalimid (bezogen von Aldrich) Die erfindungsgemäßen Beispiele weisen überraschenderweise deutlich verkürzte Brennzeiten gegenüber den Vergleichsbeispielen bei gleicher Konzentration auf.

**Tabelle 2: Zusammensetzungen in Polypropylen und Ergebnisse der Brandprüfung**

| Erfindungsgemäßes Beispiel | Zusammensetzung Flammschutzmittel | Brennzeiten als Summe der Nachbrennzeiten von 5 Prüfkörper bei 2 Beflammungen [in Sekunden] | Klassifizierung nach DIN EN 60695-11-10 |
|---|---|---|---|
| Beispiel 3 | 6% Diethylaluminiumphosphinat + 4 % Verbindung 2 | 27 | V-2 |
| Beispiel 4 | 15 % Bromiertes Polyacrylat + 5 % Verbindung 2 | 13 | V-2 |
| Beispiel 5 | 2 % Phosphonat + 8 % Verbindung 2 | 10 | V-2 |
| Beispiel 6 | 8 % Diethylaluminumphosphinat + 2 % Verbindung 3 | 27 | V-2 |
| Beispiel 7 | 6% Diethylaluminiumphosphinat + 4 % Verbindung 3 | 25 | V-2 |
| Beispiel 8 | 15 % Diethylaluminumphosphinat + 5 % Verbindung 4 | 15 | V-2 |
| Beispiel 9 | 8% Diethylaluminiumphosphinat + 2 % Verbindung 4 | 33 | V-2 |
| Beispiel 10 | 6% Diethylaluminiumphosphinat + 4 % Verbindung 4 | 33 | V-2 |
| Beispiel 11 | 8 % Aluminiumhypophosphit + 2 % Verbindung 4 | 34 | V-2 |
| Beispiel 12 | 8 % Phosphonat + 2 % Verbindung 4 | 1 | V-0 |
| Beispiel 13 | 6 % Phosphonat + 2 % Verbindung 4 | 15 | V-2 |
| Beispiel 14 | 5 % Phosphonat + 5 % Verbindung 4 | 6 | V-2 |
| Beispiel 15 | 6 % Phosphonat + 4 % Verbindung 4 | 0 | V-0 |
| Beispiel 16 | 2% Diethylaluminiumphosphinat + 8 % Verbindung 5 | 16 | V-2 |
| Beispiel 17 | 4% Diethylaluminiumphosphinat + 6 % Verbindung 5 | 26 | V-2 |
| Beispiel 18 | 8 % Phosphonat + 2 % Verbindung 5 | 2 | V-2 |
| Beispiel 19 | 6 % Phosphonat + 4 % Verbindung 5 | 0 | V-0 |
| Beispiel 20 | 15 % Diethylaluminiumphosphinat + 2 % Verbindung 6 | 35 | V-2 |
| Beispiel 21 | 6% Diethylaluminiumphosphinat + 4 % Verbindung 6 | 29 | V-2 |
| Beispiel 22 | 6 % Phosphonat + 4 % Verbindung 6 | 5 | V-2 |
| Beispiel 23 | 6 % Phosphonat + 2 % Verbindung 6 | 10 | V-2 |
| Beispiel 24 | 15 % Diethylaluminiumphosphinat + 2 % Verbindung 7 | 17 | V-2 |
| Beispiel 25 | 8% Diethylaluminiumphosphinat + 2 % Verbindung 7 | 9 | V-2 |
| Beispiel 26 | 6% Diethylaluminiumphosphinat + 4 % Verbindung 7 | 11 | V-2 |
| Beispiel 27 | 8 % Phosphonat + 2 % Verbindung 7 | 8 | V-0 |
| Beispiel 28 | 6 % Phosphonat + 4 % Verbindung 7 | 4 | V-0 |
| Beispiel 29 | 6 % Phosphonat + 2 % Verbindung 7 | 6 | V-0 |
| Beispiel 30 | 5 % Phosphonat + 5 % Verbindung 7 | 5 | V-0 |
| Beispiel 31 | 15 % Bromiertes Polyacrylat + 5 % Verbindung 7 | 16 | V-2 |
| Beispiel 32 | 15 % Diethylaluminiumphosphinat + 2 % Verbindung 8 | 57 | V-2 |
| Beispiel 33 | 8 % Phosphonat + 2 % Verbindung 8 | 0 | V-2 |
| Beispiel 34 | 6% Diethylaluminiumphosphinat + 2% Alkoxyamin + 2 % Verbindung 3 | 19 | V-2 |
| Beispiel 35 | 6% Diethylaluminiumphosphinat + 2 % Disulfid + 2 % Verbindung 3 | 59 | V-2 |

| | | | |
|---|---|---|---|
| Diethylaluminiumphosphinat = Exolit OP 1230 der Fa. Clariant SE Phosphonat = Aflammit PCO 900 der Fa. Thor GmbH Aluminiumhypophosphit = DP 111 der Fa. Velox Bromiertes Polyacrylat = FR 1025 der Fa. ICL-IP Alkoxyamin= Flamestab NOR 116 der Fa. BASF SE Disulfid= Hostanox SE 10 der Fa. Clariant SE | | | |

Beispiel 36: Analog zu den erfindungsgemäßen Beispielen 1-35 wurde ein thermoplastisches Polyurethan (Elastollan 1185A der Fa. BASF SE) mit 8 % Diethylaluminiumphosphinat und 4 % der Verbindung 3 bei 190 °C verarbeitet und bei 200 °C zu Prüfkörpern verpresst. Es wird die Klassifikation V-2 mit einer Gesamtbrenndauer (4 Prüfkörper) von 1,3 Sekunden erhalten.

Analog der Beispiele 1-35 wurden Folien aus Polypropylen (Sabic 575 P) hergestellt und mittels der Norm DIN 4102 B2 geprüft, es werden die in der Tabelle 3 zusammengestellten Ergebnisse erhalten:

**Tabelle 3: Ergebnisse von flammgeschützten Polypropylen-Folien**

| Beispiel | Zusammensetzung Flammschutzmittel | Maximale Brandhöhe [in mm] | Klassifizierung nach DIN 4102 B2 (bestanden/nicht bestanden) |
|---|---|---|---|
| Vergleichsbeispiel 3 | Ohne Flammschutzmittel | 150 | Nicht bestanden |
| Erfindungsgemäßes Beispiel 37 | 4 % Verbindung 4 | 50 | Bestanden |
| Erfindungsgemäßes Beispiel 38 | 2 % Verbindung 4 | 68 | Bestanden |
| Erfindungsgemäßes Beispiel 39 | 0.4 % Verbindung 4 + 3.6 % Phosphonat | 88 | Bestanden |
| Erfindungsgemäßes Beispiel 40 | 1 % Verbindung 4 + 2 % Phosphat | 75 | Bestanden |

| | | | |
|---|---|---|---|
| Phosphonat = Aflammit PCO 900 der Fa. Thor GmbH Phosphat= Phosphorsäure, *P,P*'-[1,1'-biphenyl]-4,4'-diyl *P,P,P*'*,P*'-tetraphenyl ester =ADK Stab FP 800 der Fa. Adeka | | | |

## Patentansprüche

1. Verwendung von organischen Oxyimiden, enthaltend mindestens ein Strukturelement der nachfolgend abgebildeten Formel I 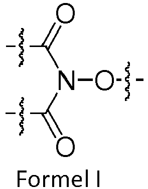 als Flammschutzmittel für Kunststoffe, wobei das organische Oxyimid Halogen-frei ist.

2. Verwendung nach Anspruch 1, **dadurch gekennzeichnet, dass** das Oxyimid ausgewählt ist aus der Gruppe bestehend aus
a) Oxyimiden, enthaltend mindestens ein Strukturelement der nachfolgenden Formel II 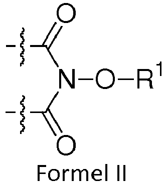 wobei R¹ für Wasserstoff oder einen gegebenenfalls substituierter Alkyl-, Cycloalkyl-, Aryl-, Heteroaryl- oder Acyl-Rest steht, sowie
b) verbrückten Oxyimiden, enthaltend mindestens ein Strukturelement der nachfolgenden Formel III 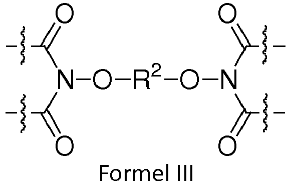 wobei R² für einen gegebenenfalls substituierten Alkylen-, Cycloalkylen-, Arylen-, Heteroarylen- oder verbrückenden Acyl-Rest steht.

3. Verwendung nach vorhergehendem Anspruch, **dadurch gekennzeichnet, dass** R² ausgewählt ist aus Resten der Gruppe bestehend aus
-(CH₂)ₙ- mit n = 1 bis 18, -CH(CH₃)-, -C(CH₃)₂-, -O-, -S-, -SO₂-,-NHCO-, -CO- sowie den nachfolgend abgebildeten Gruppen 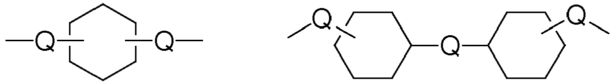 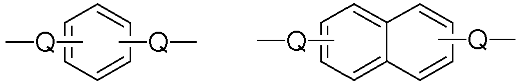 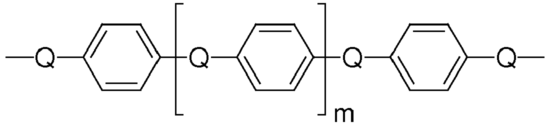 wobei
die in den zuvor abgebildeten Gruppen enthaltenen cycloaliphatischen oder aromatischen Ringsysteme unsubstituiert oder durch eine oder mehrere Alkyl- und/oder Alkoxygruppen substituiert sind,
Q bei jedem Auftreten gleich oder verschieden ist und ausgewählt ist aus der Gruppe bestehend aus einer chemischen Bindung sowie den Resten -(CH₂)ₙ- mit n = 1 bis 18, -CH(CH₃)-, -C(CH₃)₂-, -O-, -S-,-SO₂-, -NHCO-,
-CO-, und
m 0 oder 1 bis 18 ist.

4. Verwendung nach einem der vorhergehenden Ansprüche, **dadurch gekennzeichnet, dass** das organische Oxyimid eine der nachfolgenden Formeln aufweist 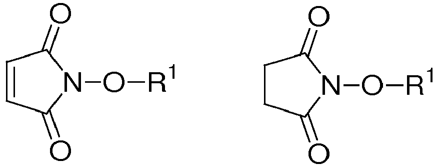 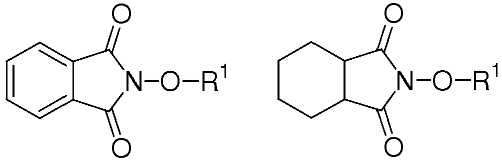 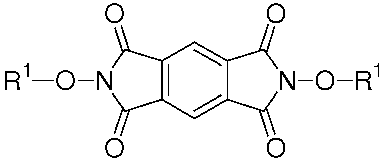 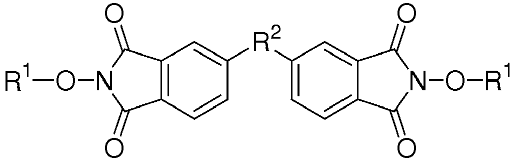 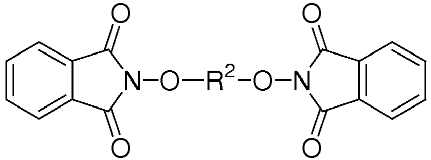 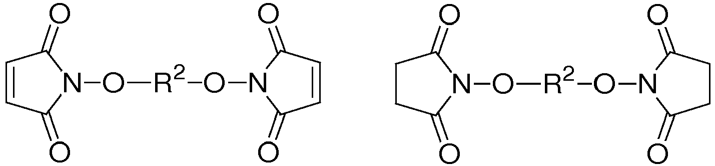 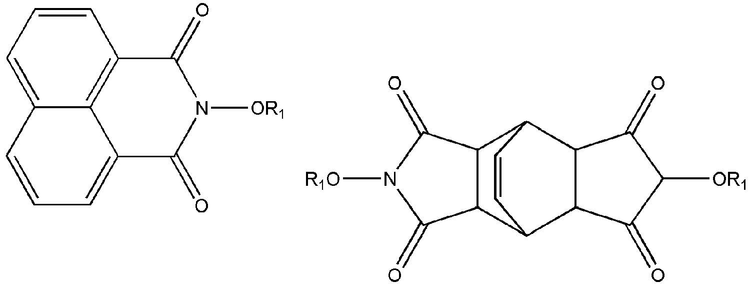 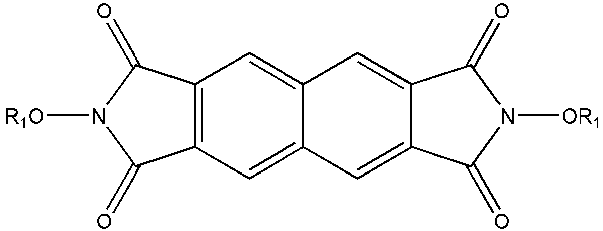 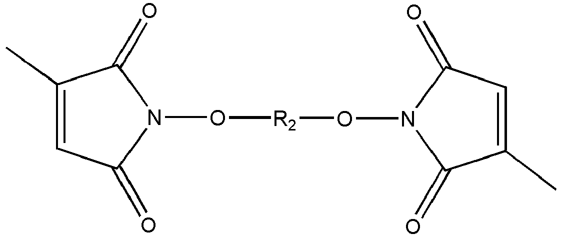 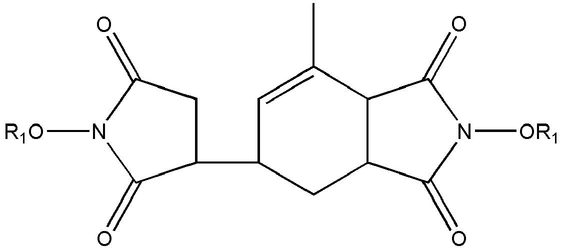 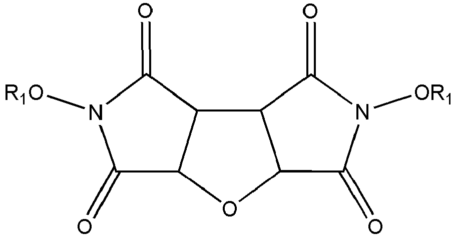 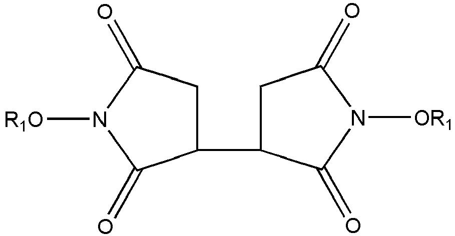 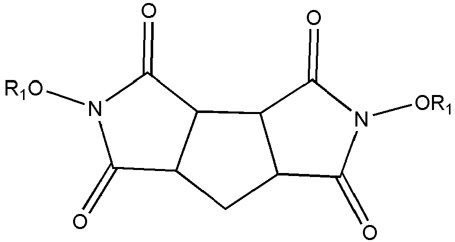 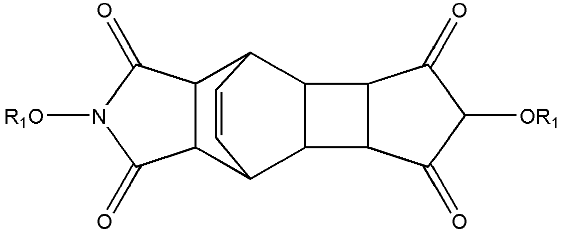 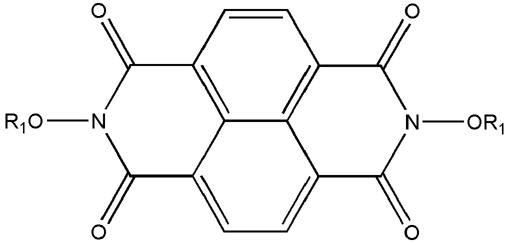 wobei jeweils R¹ und R² die oben angegebene Bedeutung aufweisen.

5. Verwendung nach einem der Ansprüche 2 bis 4, **dadurch gekennzeichnet, dass** R¹ = H oder R¹ = Acyl.

6. Verwendung nach einem der vorhergehenden Ansprüche, **dadurch gekennzeichnet, dass** die Kunststoffe thermoplastische, elastomere oder duroplastische Polymere sind, wobei die Kunststoffe bevorzugt thermoplastische Polymere sind und insbesondere ausgewählt sind aus der Gruppe bestehend aus
a) Polymere aus Olefinen oder Diolefinen wie z.B. Polyethylen (LDPE, LLDPE, VLDPE, ULDPE, MDPE, HDPE, UHMWPE), Metallocen-PE (m-PE), Polypropylen, Polyisobutylen, Poly-4-methyl-penten-1, Polybutadien, Polyisopren, Polycycloocten, Polyalkylen-Kohlenmonoxid-Copolymere, sowie Copolymere in Form von statistischen oder Blockstrukturen wie z.B. Polypropylen-Polyethylen (EP), EPM oder EPDM, EthylenVinylacetat (EVA), Ethylen-Acrylester, wie z.B. Ethylen-Butylacrylat, Ethylen-Acrylsäure und deren Salze (lonomere), sowie Terpolymere wie z.B. Ethylen-Acrylsäure-Glycidylacrylat, Pfropfpolymere wie z.B. Polypropylen-g-Maleinsäureanhydrid, Polypropylen-g-Acrylsäure, Polyethylen-g-Acrylsäure,
b) Polystyrol, Polymethylstyrol, Polyvinylnaphthalin, Styrol-Butadien (SB), Styrol-Butadien-Styrol (SBS), Styrol-Ethylen-Butylen-Styrol (SEBS), Styrol-Ethylen-Propylen-Styrol, Styrolisopren, Styrol-Isopren-Styrol (SIS), Styrol-butadienacrylnitril (ABS), Styrol-acrylnitril-acrylat (ASA), Styrol-Ethylen, Styrol-Maleinsäureanhydrid-Polymere einschl. entsprechender Pfropfcopolymere wie z.B. Styrol auf Butadien, Maleinsäureanhydrid auf SBS oder SEBS, sowie Pfropfcopolymere aus Methylmethacrylat, Styrol-Butadien und ABS (MABS),
c) halogenenthaltende Polymere wie z.B. Polyvinylchlorid (PVC), Polychloropren und Polyvinylidenchlorid (PVDC), Copolymere aus Vinylchlorid und Vinylidenchlorid oder aus Vinylchlorid und Vinylacetat, chloriertes Polyethylen, Polyvinylidenfluorid,
d) Polymere von ungesättigten Estern wie z.B. Polyacrylate und Polymethacrylate wie Polymethylmethacrylat (PMMA), Polybutylacrylat, Polylaurylacrylat, Polystearylacrylat, Polyacrylnitril, Polyacrylamide, Copolymere wie z.B. Polyacrylnitril-Polyalkylacrylat,
e) Polymere aus ungesättigten Alkoholen und Derivaten, wie z.B. Polyvinylalkohol, Polyvinylacetat, Polyvinylbutyral,
f) Polyacetale, wie z.B. Polyoxymethylen POM) oder Copolymere mit z.B. Butanal,
g) Polyphenylenoxide und Blends mit Polystyrol oder Polyamiden,
h) Polymere von cyclischen Ethern wie z.B. Polyethylenglycol, Polypropylenglycol, Polyethylenoxid, Polypropylenoxid,
i) Polyurethane, aus hydroxyterminierten Polyethern oder Polyestern und aromatischen oder aliphatischen Isocyanaten insbesondere lineare Polyurethane, Polyharnstoffe,
j) Polyamide wie z.B. Polyamid-6, 6.6, 6.10, 4.6, 4.10, 6.12, 12.12, Polyamid 11, Polyamid 12 sowie (teil-)aromatische Polyamide wie z.B. Polyphthalamide, z.B. hergestellt aus Terephthalsäure und/oder Isophthalsäure und aliphatischen Diaminen oder aus aliphatischen Dicarbonsäuren wie z.B. Adipinsäure oder Sebazinsäure und aromatischen Diaminen wie z.B. 1,4- oder 1,3-Diaminobenzol,
k) Polyimide, Polyamid-imide, Polyetherimide, Polyesterimide, Poly(ether)ketone, Polysulfone, Polyethersulfone, Polyarylsulfone, Polyphenylensulfid, Polybenzimidazole, Polyhydantoine,
l) Polyester aus aliphatischen oder aromatischen Dicarbonsäuren und Diolen oder aus Hydroxy-Carbonsäuren wie z.B. Polyethylenterephthalat (PET), Polybutylenterephthalat (PBT), Polypropylenterephthalat, Polyethylennaphthylat, Poly-1,4-dimethylolcyclohexanterephthalat, Polyhydroxybenzoat, Polyhydroxynaphthalat, Polymilchsäure,
m) Polycarbonate, Polyestercarbonate, sowie Blends wie z.B. PC/ABS, PC/PBT, PC/PET/PBT,
n) Cellulosederivate wie z.B. Cellulosenitrat, Celluloseacetat, Cellulosepropionat, Cellulosebutyrat
o) nicht-thermoplastischen oder duroplastischen Kunststoffen,
p) sowie Mischungen, Kombinationen oder Blends aus zwei oder mehr der zuvor genannten Polymere.

7. Verwendung nach einem der vorhergehenden Ansprüche, **dadurch gekennzeichnet, dass** die organischen Oxyimide in Kombination mit mindestens einem weiteren Flammschutzmittel, bevorzugt ausgewählt aus der Gruppe bestehend aus
a) anorganischen Flammschutzmitteln wie z.B. Al(OH)₃, Mg(OH)₂, AlO(OH), MgCO₃, Schichtsilikate wie z.B. Montmorillonit, nicht oder organisch modifiziert, Doppelsalze, wie z.B. Mg-Al-Silikate, POSS-(Polyhedral Oligomeric Silsesquioxane) Verbindungen, Huntit, Hydromagnesit oder Halloysit sowie Sb₂O₃, Sb₂O₅, MoO₃, Zinkstannat, Zinkhydroxystannat,
b) stickstoffhaltigen Flammschutzmitteln wie z.B. Melamin, Melem, Melam, Melon, Melaminderivate, Melaminkondensationsprodukte oder Melaminsalze, Benzoguanamin, Polyisocyanurate, Allantoin, Phosphacene, insbesondere Melamincyanurat, Melaminphosphat, Dimelaminphosphat, Melaminpyrophosphat, Melaminpolyphosphat, Melamin-Metall-Phosphate wie z.B. Melaminaluminumphosphat, Melaminzinkphosphat, Melaminmagnesiumphopsphat, sowie die entsprechenden Pyrophosphate und Polyphosphate, Poly-[2,4-(piperazin-1,4-yl)-6-(morpholin-4-yl)-1,3,5-triazin], Ammoniumpolyphosphat, Melaminborat, Melaminhydrobromid,
c) Radikalbildnern,
d) Phosphorhaltigen Flammschutzmitteln wie z.B. roter Phosphor, Phosphate wie z.B. Resorcindiphosphat, Bisphenol-A-diphosphat und ihre Oligomere, Triphenylphosphat, Ethylendiamindiphosphat, Phosphinate wie z.B. Salze der hypophosphorigen Säure und Ihrer Derivate wie Diethylaluminiumphosphinat oder Aluminiumphosphinat, Aluminiumphosphit, Aluminiumphosphonat, Phosphonatester, oligomere und polymere Derivate der Methanphosphonsäure, 9,10-dihydro-9-oxa-10-phosphorylphenanthren-10-oxid (DOPO) und deren substituierte Verbindungen.
e) Halogenhaltigen Flammschutzmitteln auf Chlor- und Brombasis wie z.B. polybrominierte Diphenyloxide, wie z.B. Decabromdiphenyloxid,Tris(3-bromo-2,2-bis(bromo-methyl)propyl-phosphat, Tris(tribromneopentyl)phosphat, Tetrabromphthalsäure, 1,2-Bis(tribromphenoxy)ethan, Hexabromcyclododecan, bromiertes Diphenylethan, Tris-(2,3-dibrompropyl)isocyanurat, Ethylen-bis(tetrabromophthalimid), Tetrabromo-bisphenol A, bromiertes Polystyrol, bromiertes Polybutadien bzw, Polystyrol-bromiertes Polybutadien-Copolymere, bromiertes Epoxidharz, Polypentabrombenzylacrylat, ggf. in Kombination mit Sb₂O₃ und/oder Sb₂O₅,
f) Boraten wie z.B. Zinkborat oder Calciumborat
g) Antidrip-Mitteln wie z.B. Polytetrafluorethylen
h) siliciumhaltigen Verbindungen wie z.B. Polyphenylsiloxane sowie Kombinationen oder Mischungen hieraus verwendet werden.

8. Verwendung nach vorhergehendem Anspruch, **dadurch gekennzeichnet, dass** die Radikalbildner ausgewählt sind aus der Gruppe bestehend aus N-Alkoxyaminen, -C-C- Radikalbildnern, Radikalbildnern mit Azogruppen (-N=N-), Radikalbildnern mit Hydrazingruppen (-NH-HN-), Radikalbildnern mit Hydrazongruppen (>C=N-NH-), Radikalbildnern mit Azingruppen (>C=N-N=C<), Radikalbildnern mit Triazengruppen (-N=N-N<), Radikalbildnern mit Disulfid- bzw. Polysulfidgrupen (-S-S-), Radikalbildnern mit Thiolgruppen (-S-H), Thiuramsulfid, Dithiocarbamaten, Mercaptobenzthiazol und Sulfenamiden, wobei die Radikalbildner bevorzugt ausgewählt sind aus der Gruppe bestehend aus
a) N-Alkoxylaminen gemäß der nachfolgend abgebildeten Strukturformel 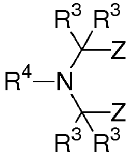 wobei
R³ für Wasserstoff oder einen gegebenenfalls substituierter Alkyl-, Cycloalkyl-, Aryl-, Heteroaryl- oder Acyl-Rest steht, insbesondere ein C1 bis C4- Alkylrest ist,
R⁴ für einen Alkoxy-, Aryloxy-, Cycloalkoxy-, Aralkoxy- oder Acyloxy-Rest steht,
Z für Wasserstoff oder einen gegebenenfalls substituierter Alkyl-, Cycloalkyl-, Aryl-, Heteroaryl- oder Acyl-Rest steht, wobei die beiden Reste Z auch einen geschlossenen Ring bilden können, der ggf. durch Ester-, Ether-, Amin, Amid, Carboxy- oder Urethangruppen substituiert sein kann,
b) Azo-Verbindungen gemäß der nachfolgend abgebildeten Strukturformeln 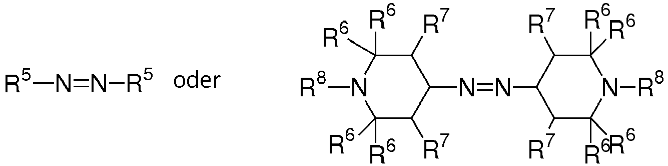 wobei
R⁵ einen Alkly-, Cycloalkyl- oder Arylrest bedeutet,
R⁶ bei jedem Auftreten gleich oder verschieden ist und einen linearen oder verzweigten Alkylrest bedeutet,
R⁷ bei jedem Auftreten gleich oder verschieden ist und Wasserstoff oder einen linearen oder verzweigten Alkylrest bedeutet, und
R⁸ bei jedem Auftreten gleich oder verschieden ist und einen Alkyl, Alkoxy-, Aryloxy- Cycloalkyloxy-, Aralkoxy oder Acyloxyrest bedeutet,
c) Dicumylene gemäß der nachfolgend abgebildeten Strukturformel 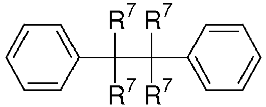 wobei R⁷ die zuvor angegebene Bedeutung aufweist, bevorzugt Methyl ist,
d) und/oder Polycumylene gemäß der nachfolgend abgebildeten Strukturformel 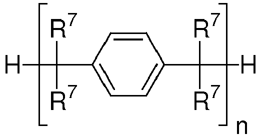 wobei R⁷ die zuvor angegebene Bedeutung aufweist, bevorzugt Methyl ist, und 2 < n < 100.

9. Verwendung nach einem der beiden vorhergehenden Ansprüche, **dadurch gekennzeichnet, dass** die organischen Oxyimide und das mindestens eine weitere Flammschutzmittel in einem Gewichtsverhältnis von 99:1 bis 1:99, bevorzugt von 5 : 95 bis 50 : 50, besonders bevorzugt von 10 : 90 bis 30 : 70 verwendet werden.

10. Verwendung nach einem der vorhergehenden Ansprüche, **dadurch gekennzeichnet, dass** die organischen Oxyimide, bezogen auf die Kunststoffe, zu 0,01 bis 30 Gew.-%, bevorzugt zu 0,1 bis 20 Gew.-%, besonders bevorzugt zu 1 bis 10 Gew.-% eingesetzt werden.

11. Flammgeschützte Kunststoffzusammensetzung, enthaltend oder bestehend aus
a) 50 bis 98 Gew.-Teilen, bevorzugt 70 bis 95 Gew.-Teilen mindestens eines Kunststoffes, insbesondere mindestens eines thermoplastischen Polymers,
b) 1 bis 25 Gew.-Teilen, bevorzugt 2,5 bis 15 Gew.-Teilen mindestens eines organischen Oxyimids, enthaltend mindestens ein Strukturelement der nachfolgend abgebildeten Formel I 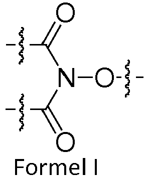 wobei das organische Oxyimid Halogen-frei ist,
c) 1 bis 25 Gew.-Teilen, bevorzugt 2,5 bis 15 Gew.-Teilen mindestens eines weiteren Flammschutzmittels, wobei bevorzugt zusätzlich
d) bis 40 Gew.-Teile mindestens eines Verstärkungs- oder Füllstoffes und/oder
e) bis 5 Gew.-Teile mindestens eines Additivs aus der Klasse der phenolischen Antioxidantien, Phosphite, Säurefänger, gehinderten Amine, Dispergiermittel sowie Kombinationen hiervon enthalten sind.

12. Flammgeschützte Kunststoffzusammensetzung nach einem der beiden vorhergehenden Ansprüche, **dadurch gekennzeichnet, dass** Zusatzstoffe ausgewählt aus der Gruppe bestehend aus UV-Absorber, der Lichtstabilisatoren, der Stabilisatoren, der Hydroxylamine, der Benzofuranone, der Metalldesaktivatoren, der Füllstoffdesaktivatoren, der Nukleierungsmittel, Schlagzähigkeitsverbesserer, Weichmacher, Gleitmittel, Rheologiemodifikatoren, Verarbeitungshilfsmittel, Pigmente, Farbstoffe, optische Aufheller, antimikrobielle Wirkstoffe, Antistatika, Slipmittel, Antiblockmittel, Kopplungsmittel, Dispergiermittel, Kompatibilisatoren, Sauerstofffänger, Säurefänger, Markierungsmittel oder Antifoggingmittel enthalten sind.

13. Verfahren zur Herstellung einer flammgeschützten Kunststoffzusammensetzung nach einem der Ansprüche 11 bis 12, bei dem
a) 1 bis 25 Gew.-Teile, bevorzugt 2,5 bis 15 Gew.-Teile mindestens eines organischen Oxyimids, enthaltend mindestens ein Strukturelement der nachfolgend abgebildeten Formel I 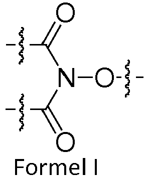 wobei das organische Oxyimid Halogen-frei ist,
b) vor, nach oder gleichzeitig mit 1 bis 25 Gew.-Teilen, bevorzugt 2,5 bis 15 Gew.-Teilen mindestens eines weiteren Flammschutzmittels
in 50 bis 98 Gew.-Teile, bevorzugt 70 bis 95 Gew.-Teile mindestens eines Kunststoffes, insbesondere mindestens eines thermoplastischen Polymers eingebracht wird.

14. Formmasse, Formteil, Lack oder Beschichtung, herstellbar aus einer flammgeschützten Kunststoffzusammensetzung nach einem der Ansprüche 11 bis 12, insbesondere in Form von Spritzgussteilen, Folien oder Filmen, Beschichtungen oder Lacken, Schäumen, Fasern, Kabeln und Rohren, Profilen, Bändchen, Membranen, wie z.B. Geomembranen, Klebstoffen, die über Extrusion, Spritzguss, Blasformen, Kalandrieren, Pressverfahren, Spinnprozesse oder Streich- und Beschichtungsprozesse hergestellt werden z.B. für die Elektro- und Elektronikindustrie, Bauindustrie, Transportindustrie, für Haushalts- und Elektrogeräte, Fahrzeugteile, Konsumartikel, Möbel, Textilien.

15. Flammschutzzusammensetzung bestehend aus einem organischen Oxyimid, enthaltend mindestens ein Strukturelement der nachfolgend abgebildeten Formel I, 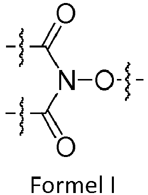 und mindestens einem weiteren Flammschutzmittel, wobei das organische Oxyimid Halogen-frei ist.

## Claims

1. Use of organic oxyimides, comprising at least one structural element of the subsequently illustrated formula I 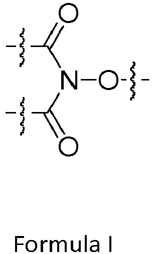 as flame retardant for plastic materials, the organic oxyimide being halogen-free.

2. Use according to claim 1, **characterised in that** the oxyimide is selected from the group consisting of
a) oxyimides, comprising at least one structural element of the subsequent formula II, 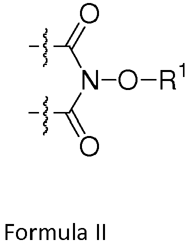 R¹ standing for hydrogen or a possibly substituted alkyl-, cycloalkyl-, aryl-, heteroaryl- or acyl radical, and also
b) bridged oxyimides, comprising at least one structural element of the subsequent formula III, 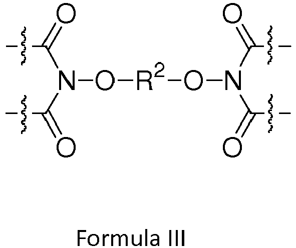 R² standing for a possibly substituted akylene-, cycloalkylene-, arylene-, heteroarylene- or bridging acyl radical.

3. Use according to the preceding claim, **characterised in that** R² is selected from radicals of the group consisting of
-(CH₂)n- with n = 1 to 18, -CH(CH₃)-, -C(CH₃)₂-, -O-, -S-, -SO₂-, -NHCO-, -CO- and also the subsequently illustrated groups, 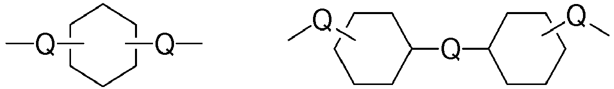 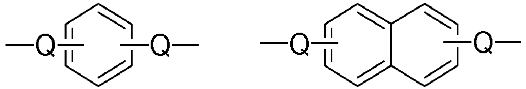 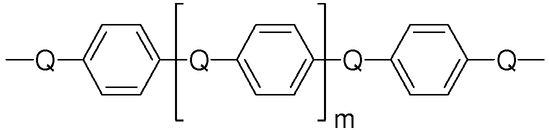 the cycloaliphatic or aromatic ring systems contained in the previously illustrated groups being unsubstituted or substituted by one or more alkyl- and/or alkoxy groups,
Q upon each occurrence, being the same or different and being selected from the group consisting of a chemical bond and also the radicals-(CH₂)ₙ- with n = 1 to 18, -CH(CH₃)-, -C(CH₃)₂-, -O-, -S-, -SO₂-, -NHCO-,
-CO-, and
m being 0 or 1 to 18.

4. Use according to one of the preceding claims, **characterised in that** the organic oxyimide has one of the following formulae, 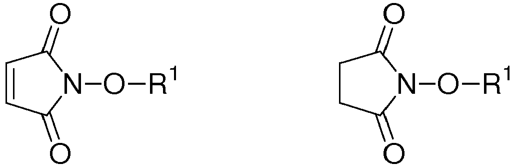 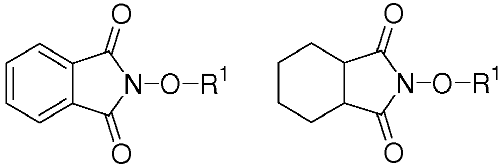 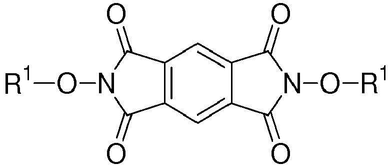 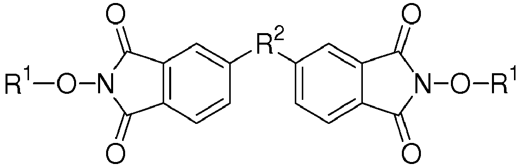 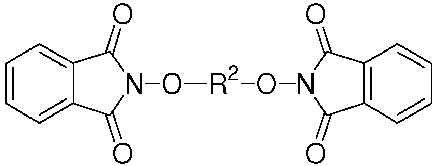 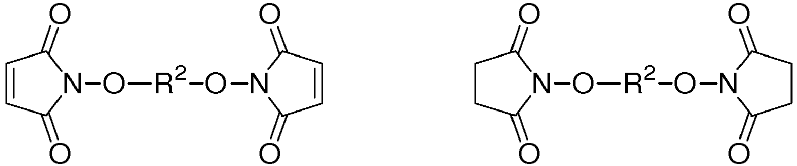 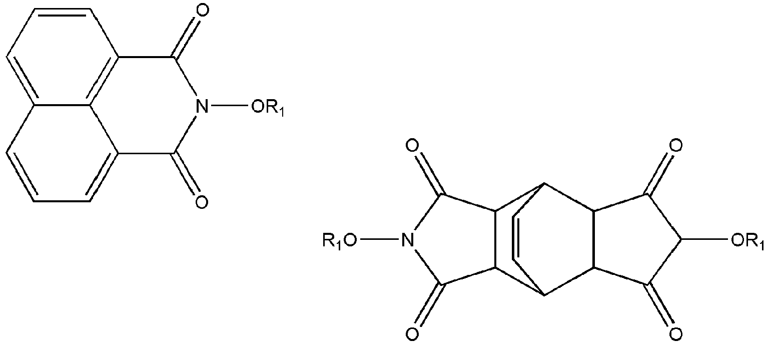 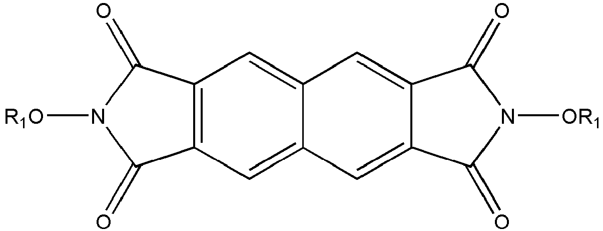 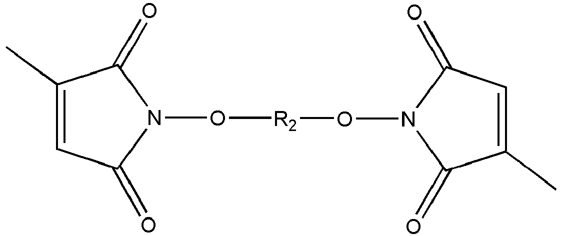 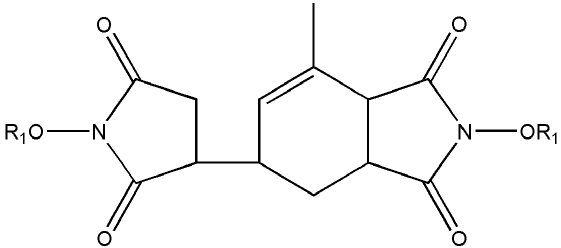 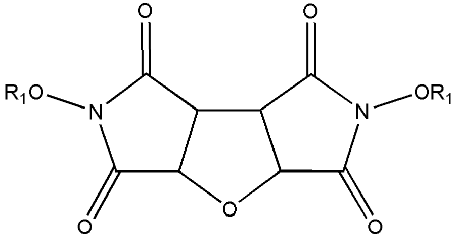 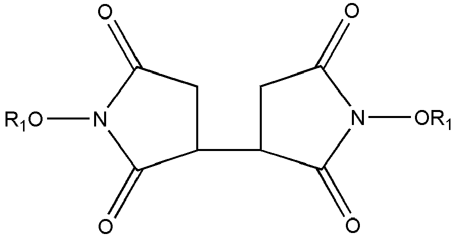 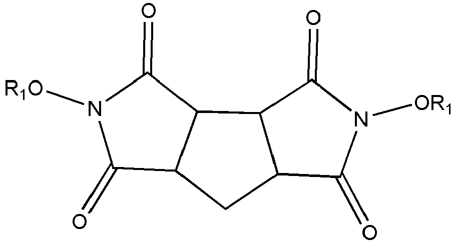 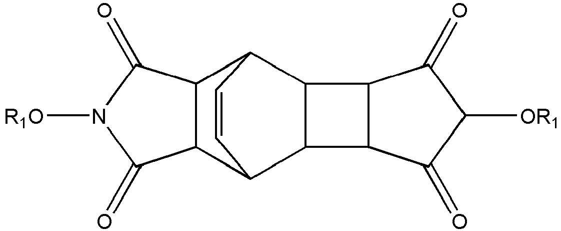 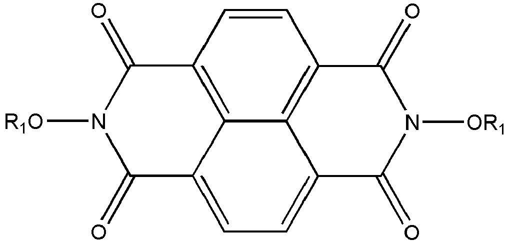 respectively R¹ and R² having the above-indicated meaning.

5. Use according to one of the claims 2 to 4, **characterised in that** R¹ = H or R¹ = acyl.

6. Use according to one of the preceding claims, **characterised in that** the plastic materials are thermoplastic, elastomeric or duroplastic polymers, the plastic materials preferably being thermoplastic polymers and in particular selected from the group consisting of
a) polymers made of olefins or diolefins, such as e.g. polyethylene (LDPE, LLDPE, VLDPE, ULDPE, MDPE, HDPE, UHMWPE), metallocene-PE (m-PE), polypropylene, polyisobutylene, poly-4-methylpentene-1, polybutadiene, polyisoprene, polycyclooctene, polyalkylene-carbon monoxide copolymers, and also copolymers in the form of stochastic or block structures such as e.g. polypropylene-polyethylene (EP), EPM or EPDM, ethylene-vinyl acetate (EVA), ethylene-acrylic ester, such as e.g. ethylene-butyl acrylate, ethylene-acrylic acid and the salts thereof (ionomers), and also terpolymers, such as e.g. ethylene-acrylic acid-glycidylacrylate, graft polymers, such as e.g. polypropylene-g-maleic anhydride, polypropylene-g-acrylic acid, polyethylene-g-acrylic acid,
b) polystyrene, polymethylstyrene, polyvinylnaphthalene, styrene-butadiene (SB), styrene-butadiene-styrene (SBS), styrene-ethylene-butylene-styrene (SEBS), styene-ethylene-propylene-styrene, styrene-isoprene, styrene-isoprene-styrene (SIS), styrene-butadiene-acrylonitrile (ABS), styreneacrylonitrile-acrylate (ASA), styrene-ethylene, styrene-maleic anhydride polymers incl. corresponding graft copolymers, such as e.g. styrene on butadiene, maleic anhydride on SBS or SEBS, and also graft copolymers made of methylmethacrylate, styrene-butadiene and ABS (MABS),
c) halogen-containing polymers, such as e.g polyvinyl chloride (PVC), polychloroprene and polyvinylidene chloride (PVDC), copolymers made of vinyl chloride and vinylidene chloride or made of vinyl chloride and vinyl acetate, chlorinated polyethylene, polyvinylidene fluoride,
d) polymers of unsaturated esters, such as e.g polyacrylates and polymethacrylates, such as polymethylmethacrylate (PMMA), polybutylacrylate, polylaurylacrylate, polystearylacrylate, polyacrylonitrile, polyacrylamides, copolymers, such as e.g. polyacrylonitrile-polyalkylacrylate,
e) polymers made of unsaturated alcohols and derivatives, such as e.g. polyvinyl alcohol, polyvinyl acetate, polyvinyl butyral,
f) polyacetals, such as e.g. polyoxymethylene (POM) or copolymers with e.g. butanal,
g) polyphenylene oxides and blends with polystyrene or polyamides,
h) polymers of cyclic ethers, such as e.g. polyethylene glycol, polypropylene glycol, polyethylene oxide, polypropylene oxide,
i) polyurethanes, made of hydroxy-terminated polyethers or polyesters and aromatic or aliphatic isocyanates, in particular linear polyurethanes, polyureas,
j) polyamides, such as e.g. polyamide 6, 6.6, 6.10, 4.6, 4.10, 6.12, 12.12, polyamide 11, polyamide 12 and also (partially) aromatic polyamides, such as e.g. polyphthalamides, e.g. produced from terephthalic acid and/or isophthalic acid and aliphatic diamines or from aliphatic dicarboxylic acids, such as e.g. adipic acid or sebacic acid and aromatic diamines, such as e.g. 1,4- or 1,3-diaminobenzene,
k) polyimides, polyamideimides, polyetherimides, polyesterimides, poly(ether)ketones, polysulphones, polyethersulphones, polyarylsulphones, polyphenylenesulphide, polybenzimidazoles, polyhydantoins,
l) polyesters made of aliphatic or aromatic dicarboxylic acids and diols or made of hydroxycarboxylic acids, such as e.g. polyethylene terephthalate (PET), polybutylene terephthalate (PBT), polypropylene terephthalate, polyethylene naphthylate, poly-1,4-dimethylolcyclohexane terephthalate, polyhydroxybenzoate, polyhydroxynaphthalate, polylactic acid,
m) polycarbonates, polyester carbonates, and also blends, such as e.g. PC/ABS, PC/PBT, PC/PET/PBT,
n) cellulose derivatives, such as e.g. cellulose nitrate, cellulose acetate, cellulose propionate, cellulose butyrate,
o) non-thermoplastic or duroplastic plastic materials,
p) and also mixtures, combinations or blends made of two or more of the previously mentioned polymers.

7. Use according to one of the preceding claims, **characterised in that** the organic oxyimides are used in combination with at least one further flame retardant, preferably selected from the group consisting of
a) inorganic flame retardants, such as e.g. Al(OH)₃, Mg(OH)₂, AlO(OH), MgCO₃, layer silicates, such as e.g. montmorillonite, non- or organically modified, double salts such as e.g. Mg-Al-silicates, POSS-(Polyhedral Oligomeric Silsesquioxane) compounds, huntite, hydromagnesite or halloysite and also Sb₂O₃, Sb₂O₅, MoO₃, zinc stannate, zinc hydroxystannate,
b) nitrogen-containing flame retardants, such as e.g. melamine, melem, melam, melon, melamine derivatives, melamine condensation products or melamine salts, benzoguanamine, polyisocyanurates, allantoin, phosphacenes, in particular melamine cyanurate, melamine phosphate, dimelamine phosphate, melamine pyrophosphate, melamine polyphosphate, melamin-metal-phosphates, such as e.g. melamine aluminum phosphate, melamine zinc phosphate, melamine magnesium phosphate, and also the corresponding pyrophosphates and polyphosphates, poly-[2,4-(piperazin-1,4-yl)-6-(morpholin-4-yl)-1,3,5-triazine], ammonium polyphosphate, melamine borate, melamine hydrobromide,
c) radical formers,
d) phosphorus-containing flame retardants, such as e.g. red phosphorus, phosphates, such as e.g. resorcin diphosphate, bisphenol-A-diphosphate and the oligomers thereof, triphenylphosphate, ethylene diamine diphosphate, phosphinates, such as e.g. salts of hypophosphorous acid and derivatives thereof, such as diethylaluminium phosphinate or aluminium phosphinate, aluminium phosphite, aluminium phosphonate, phosphonate ester, oligomeric and polymeric derivatives of methane phosphonic acid, 9,10-dihydro-9-oxa-10-phosphorylphenanthrene-10-oxide (DOPO) and the substituted compounds thereof,
e) halogen-containing flame retardants based on chlorine and bromine, such as e.g polybrominated diphenyloxides, such as e.g. decabromodiphenyl oxide, tris(3-bromo-2,2-bis(bromomethyl)propyl phosphate, tris(tribromoneopentyl)phosphate, tetrabromophthalic acid, 1,2-bis(tribromophenoxy)ethane, hexabromocyclododecane, brominated diphenylethane, tris-(2,3-dibromopropyl)isocyanurate, ethylene-bis(tetrabromophthalimide), tetrabromobisphenol A, brominated polystyrene, brominated polybutadiene or polystyrene-brominated polybutadiene copolymers, brominated epoxy resin, polypentabromobenzyl acrylate, possibly in combination with Sb₂O₃ and/or Sb₂O₅,
f) borates, such as e.g. zinc borate or calcium borate,
g) antidrip agents, such as e.g. polytetrafluorethylene,
h) silicon-containing compounds, such as e.g. polyphenylsiloxanes
and also combinations or mixtures thereof.

8. Use according to the preceding claim, **characterised in that** the radical formers are selected from the group consisting of N-alkoxyamines, -C-C- radical formers, radical formers with azo groups (-N=N-), radical formers with hydrazine groups (-NH-HN-), radical formers with hydrazone groups (>C=N-NH-), radical formers with azine groups (>C=N-N=C<), radical formers with triazene groups (-N=N-N<), radical formers with disulphide- or polysulphide groups (-S-S), radical formers with thiol groups (-S-H), thiuram sulphide, dithiocarbamates, mercaptobenzothiazole and sulphene amides, the radical formers preferably being selected from the group consisting of
a) N-alkoxyamines according to the subsequently illustrated structural formula, 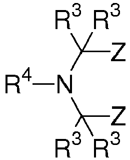
R³ standing for hydrogen or a possibly substituted alkyl, cycloalkyl-, aryl-, heteroaryl- or acyl radical, in particular being a C1 to C4 alkyl radical,
R⁴ standing for an alkoxy-, aryloxy-, cycloalkoxy-, aralkoxy- or acyloxy radical,
Z standing for hydrogen or a possibly substituted alkyl, cycloalkyl-, aryl-, heteroaryl- or acyl radical, the two radicals Z also being able to form a closed ring which can be substituted possibly by ester-, ether-, amine-, amide-, carboxy- or urethane groups,
b) azo compounds according to the subsequently illustrated structural formulae,
R⁵-N=N-R⁵
or 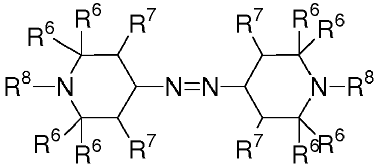
R⁵ meaning an alkyl-, cycloalkyl- or aryl radical,
R⁶ upon each occurrence, being the same or different and meaning a linear or branched alkyl radical,
R⁷ upon each occurrence, being the same or different and meaning hydrogen or a linear or branched alkyl radical, and
R⁸ upon each occurrence, being the same or different and meaning an alkyl-, alkoxy-, aryloxy-, cycloalkyloxy-, aralkoxy or acyloxy radical,
c) dicumylene according to the subsequently illustrated structural formula, 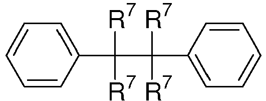 R⁷ having the previously indicated meaning, preferably being methyl,
d) and/or polycumylene according to the subsequently illustrated structural formula, 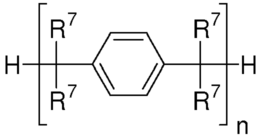 R⁷ having the previously indicated meaning, preferably being methyl, and 2 < n < 100.

9. Use according to one of the two preceding claims, **characterised in that** the organic oxyimides and the at least one further flame retardant are used in a weight ratio of 99 : 1 to 1 : 99, preferably of 5 : 95 to 50 : 50, particularly preferred of 10 : 90 to 30 : 70.

10. Use according to one of the preceding claims, **characterised in that** the organic oxyimides, relative to the plastic materials, are used at 0.01 to 30% by weight, preferably at 0.1 to 20% by weight, particularly preferred at 1 to 10% by weight.

11. Flame-retardant plastic material composition, comprising or consisting of
a) 50 to 98 parts by weight, preferably 70 to 95 parts by weight, of at least one plastic material, in particular of at least one thermoplastic polymer,
b) 1 to 25 parts by weight, preferably 2.5 to 15 parts by weight, of at least one organic oxyimide, comprising at least one structural element of the subsequently illustrated formula I, 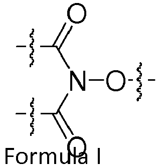 the organic oxyimide being halogen-free,
c) 1 to 25 parts by weight, preferably 2.5 to 15 parts by weight, of at least one further flame retardant, wherein preferably
further
a) up to 40 parts by weight of at least one reinforcing- or filling material and/or
b) up to 5 parts by weight of at least one additive from the class of phenolic antioxidants, phosphites, acid collectors, hindered amines, dispersants and also combinations hereof
are contained.

12. Flame-retardant plastic material composition according to one of the two preceding claims, **characterised in that** there are contained additives selected from the group consisting of UV absorbers, light stabilisers, stabilisers, hydroxylamines, benzofuranones, metal deactivators, filler deactivators, nucleation agents, impact strength enhancers, plasticisers, lubricants, rheology modifiers, processing aids, pigments, colourants, optical brighteners, antimicrobial active substances, antistatic agents, slip agents, antiblocking agents, coupling means, dispersants, compatibilisers, oxygen collectors, acid collectors, marking means or anti-fogging means.

13. Method for the production of a flame-retardant plastic material composition according to one of the claims 11 to 12, in which
a) 1 to 25 parts by weight, preferably 2.5 to 15 parts by weight, of at least one organic oxyimide, comprising at least one structural element of the subsequently illustrated formula I 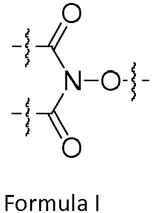 the organic oxyimide being halogen-free,
b) before, after or at the same time, with 1 to 25 parts by weight, preferably 2.5 to 15 parts by weight, of at least one further flame retardant being incorporated in 50 to 98 parts by weight, preferably 70 to 95 parts by weight of at least one plastic material, in particular of at least one thermoplastic polymer.

14. Moulding compound, moulded part, paint or coating producible from a flame-retardant plastic material composition according to one of the claims 11 to 12 , in particular in the form of injection moulded parts, foils or films, coatings or paints, foams, fibres, cables and pipes, profiles, strips, membranes, such as e.g. geomembranes, adhesives, which are produced by extrusion, injection moulding, blow-moulding, calendering, pressing processes, spinning processes or brushing and coating processes, e.g. for the electrical and electronic industry, construction industry, transport industry, for household and electrical appliances, vehicle parts, consumer articles, furniture, textiles.

15. Flame-retardant composition consisting of an organic oxyimide, comprising at least one structural element of the subsequently illustrated formula I 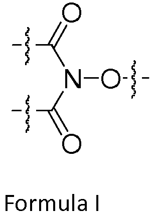 and at least one further flame retardant, the organic oxyimide being halogen-free.

## Revendications

1. Utilisation d'oxyimides organiques contenant au moins un élément structural ayant la formule I représentée ci-après 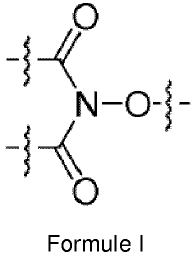 en tant qu'agent retardateur de flamme pour matières plastiques, l'oxyimide organique étant non halogéné.

2. Utilisation selon la revendication 1, **caractérisée en ce que** l'oxyimide est choisi dans le groupe consistant en :
a) les oxyimides contenant au moins un élément structural ayant la formule II ci-après 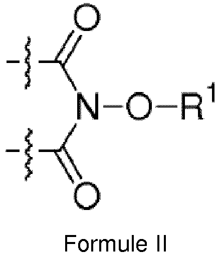 dans laquelle R¹ représente un atome d'hydrogène ou un radical alkyle, cycloalkyle, aryle, hétéroaryle ou acyle éventuellement substitué, ainsi que
b) les oxyimides pontés, contenant au moins un élément structural ayant la formule III ci-après 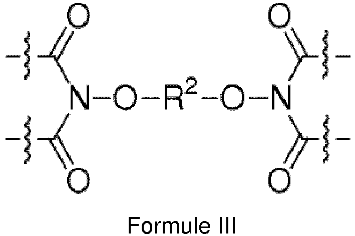 dans laquelle R² représente un radical alkylène, cycloalkylène, arylène, hétéroarylène ou acyle ponté, éventuellement substitué.

3. Utilisation selon la revendication précédente, **caractérisé en ce que** R² est choisi parmi les radicaux du groupe consistant en -(CH₂)ₙ-, dans laquelle n = 1 à 18, -CH(CH₃)-, -CH(CH₃)₂-, -O-, -S-, -SO₂-, -NHCO-, -CO-, ainsi que parmi les groupes représentés ci-après 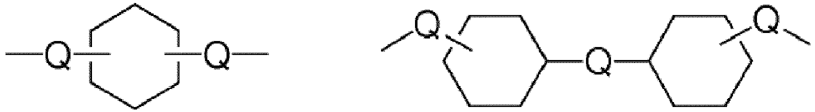 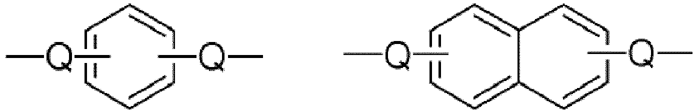 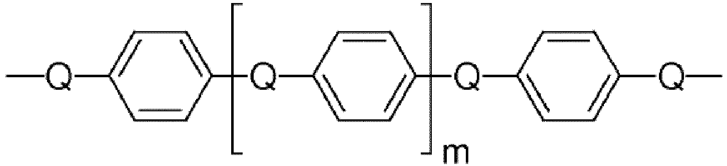 dans lesquels
les systèmes cycliques cycloaliphatiques ou aromatiques contenus dans les groupes représentés ci-après sont non substitués, ou substitués par un ou plusieurs groupes alkyle et/ou alcoxy,
Q à chaque occurrence, est identique ou différent et est choisi dans le groupe consistant en une liaison chimique, ainsi qu'en les radicaux -(CH₂)ₙ-, dans laquelle n = 1 à 18, -CH(CH₃)-, -CH(CH₃)₂-, -O-, -S-, -SO₂-, -NHCO-, -CO-, et
m vaut 0 ou 1 à 18.

4. Utilisation selon l'une des revendications précédentes, **caractérisée en ce que** l'oxyimide organique présente l'une des formules suivantes : 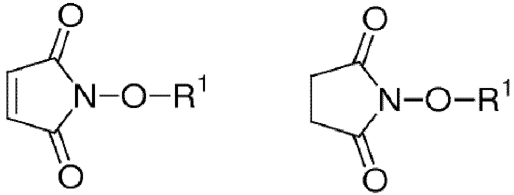 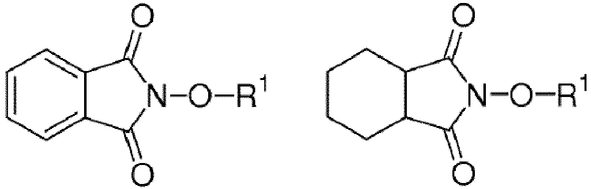 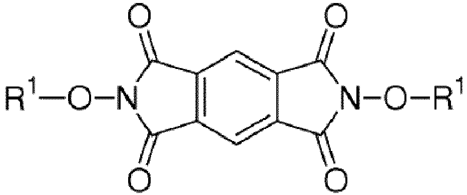 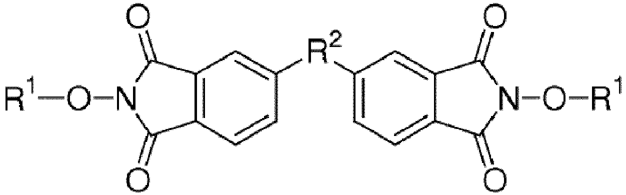 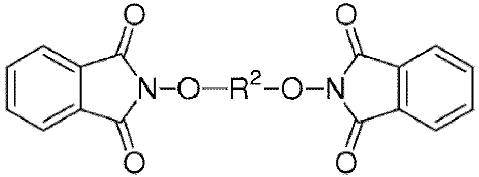 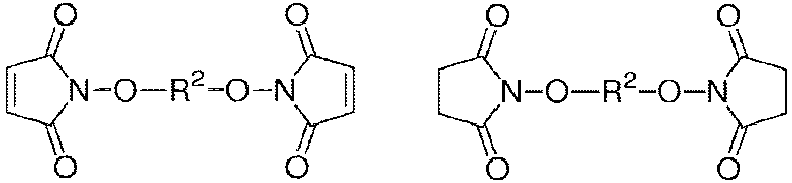 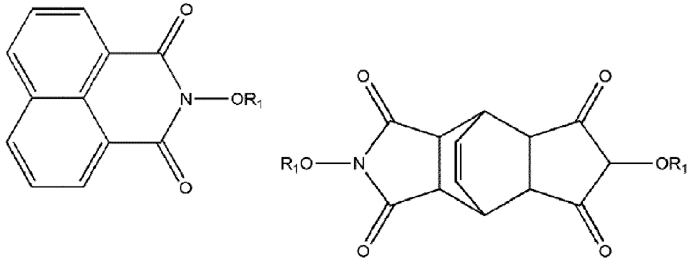 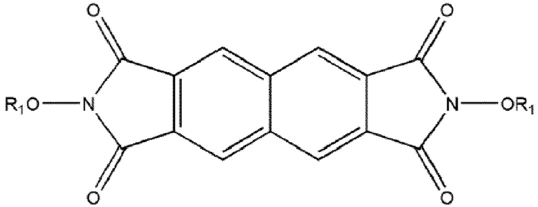 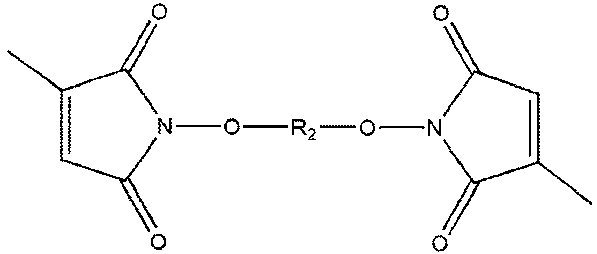 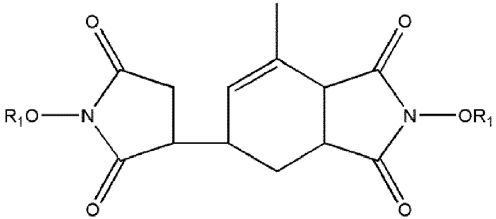 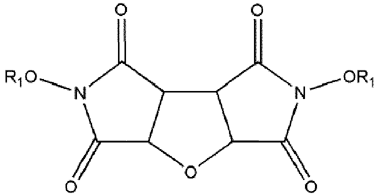 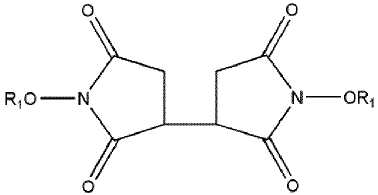 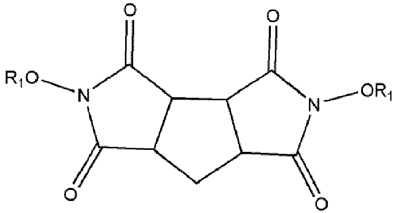 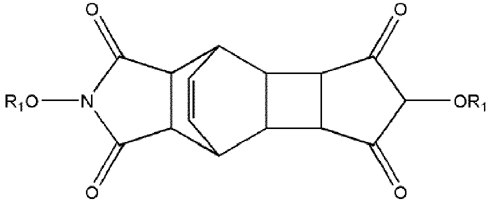 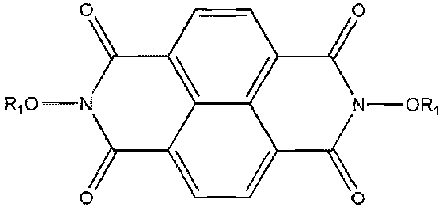 dans chacune desquelles R¹ et R² ont les significations données plus haut.

5. Utilisation selon l'une des revendications 2 à 4, **caractérisée en ce que** R¹ est H ou R¹ est un groupe acyle.

6. Utilisation selon l'une des revendications précédentes, **caractérisée en ce que** les plastiques sont des polymères thermoplastiques, élastomères ou thermodurcissables, les plastiques étant de préférence des polymères thermoplastiques et étant en particulier choisis dans le groupe consistant en
a) les polymères d'oléfines ou de dioléfines, tels que par exemple le polyéthylène (PEBD, PEBDL, VLDPE, ULDPE, MDPE, PEHD, UHMWPE), le métallocène-PE (m-PE), le polypropylène, le polyisobutylène, le poly-4-méthyl-pentène-1, le polybutadiène, le polyisoprène, le polycyclooctène, les copolymères polyalkylène-monoxyde de carbone, ainsi que les copolymères sous forme de structures statistiques ou en blocs, tels que par exemple le polypropylène-polyéthylène (EP), l'EPM ou l'EPDM, l'éthylène-acétate de vinyle (EVA), les éthylène-esters de l'acide acrylique tels que par exemple l'éthylène-acrylate de butyle, l'éthylène-acide acrylique et les sels (ionomères) de ceux-ci, ainsi que les terpolymères tels que par exemple l'éthylène-acide acrylique-acrylate de glycidyle, les polymères greffés tels que par exemple le polypropylène-greffé-anhydride maléique, le polypropylène-greffé-acide acrylique, le polyéthylène-greffé-acide acrylique,
b) le polystyrène, le polyméthylstyrène, le polyvinylnaphtalène, les polymères styrène-butadiène (SB), styrène-butadiène-styrène (SBS), styrène-éthylène-butylène-styrène (SEBS), styrène-éthylène-propylène-styrène, styrène-isoprène, styrène-isoprène-styrène (SIS), styrène-butadiène-acrylonitrile (ABS), styrène-acrylonitrile-acrylate (ASA), styrène-éthylène, styrène-anhydride maléique, y compris les copolymères greffés correspondants tels que par exemple styrène sur butadiène, anhydride maléique sur SBS ou SEBS, ainsi que les copolymères greffés constitués de méthacrylate de méthyle, de styrène-butadiène et d'ABS (MABS),
c) les polymères halogénés tels que par exemple le poly(chlorure de vinyle) (PVC), le polychloroprène et le poly(chlorure de vinylidène) (PVDC), les copolymères de chlorure de vinyle et de chlorure de vinylidène ou de chlorure de vinyle et d'acétate de vinyle, le polyéthylène chloré, le poly(fluorure de vinylidène),
d) les polymères d'esters insaturés tels que par exemple les polyacrylates et les polyméthacrylates tels que le poly(méthacrylate de méthyle) (PMMA), le poly(acrylate de butyle), le poly(acrylate de lauryle), le poly(acrylate de stéaryle), le polyacrylonitrile, les polyacrylamides, les copolymères tels que par exemple le polyacrylonitrile-poly(acrylate d'alkyle),
e) les polymères d'alcools insaturés et de leurs dérivés, tels que par exemple le poly(alcool vinylique), le poly(acétate de vinyle), le polyvinylbutyral,
f) les polyacétals, tels que par exemple le polyoxyméthylène (POM) ou les copolymères avec par exemple le butanal,
g) les poly(oxydes de phénylène) et les mélanges avec le polystyrène ou les polyamides,
h) les polymères d'éthers cycliques tels que le polyéthylèneglycol, le polypropylèneglycol, le poly(oxyde d'éthylène), le poly(oxyde de propylène),
i) les polyuréthannes, obtenus à partir de polyéthers ou de polyesters à terminaison hydroxy et d'isocyanates aromatiques ou aliphatiques, en particulier les polyuréthannes linéaires, les polyurées,
j) les polyamides tels que par exemple les polyamides-6,6.6, 6.10, 4.6, 4.10, 6.12, 12.12, le polyamide 11, le polyamide 12, ainsi que les polyamides (partiellement) aromatiques tels que par exemple les polyphtalamides, fabriqués par exemple à partir d'acide téréphtalique et/ou d'acide isophtalique et de diamines aliphatiques, ou à partir d'acides dicarboxyliques aliphatiques tels que par exemple l'acide adipique ou l'acide sébacique et de diamines aromatiques telles que par exemple le 1,4- ou le 1,3-diaminobenzène,
k) les polyimides, les polyamide-imides, les polyétherimides, les polyesterimides, les poly(éther)cétones, les polysulfones, les polyéthersulfones, les polyarylsulfones, le poly(sulfure de phénylène), les polybenzimidazoles, les polyhydantoïnes,
l) les polyesters obtenus à partir d'acides dicarboxyliques aliphatiques ou aromatiques et de diols ou d'acides hydroxycarboxyliques tels que par exemple le poly(téréphtalate d'éthylène) (PET), le poly(téréphtalate de butylène) (PBT), le poly(téréphtalate de propylène), le poly(naphtylate d'éthylène), le poly(téréphtalate de 1,4-diméthylolcyclohexane), le poly(hydroxybenzoate), le poly(hydroxynaphtalate), le poly(acide lactique),
m) les polycarbonates, les polyestercarbonates, ainsi que les mélanges tels que par exemple le PC/ABS, le PC/PBT, le PC/PET/PBT,
n) les dérivés de la cellulose tels que par exemple le nitrate de cellulose, l'acétate de cellulose, le propionate de cellulose, le butyrate de cellulose,
o) les matières plastiques non thermoplastiques ou thermodurcissables,
p) ainsi que les mélanges, combinaisons ou mélanges mécaniques de deux des polymères mentionnés ci-dessus, ou plus.

7. Utilisation selon l'une des revendications précédentes, **caractérisée en ce que** les oxyimides organiques sont utilisés en combinaison avec au moins un retardateur de flamme supplémentaire, de préférence choisi dans le groupe consistant en
a) les retardateurs de flamme inorganiques tels que Al(OH)₃, Mg(OH)₂, AlO(OH), MgCO₃, les phyllosilicates tels que par exemple la montmorillonite, non modifiée ou à modification organique, les sels doubles tels que par exemple les silicates de Mg-Al, les composés POSS-(silsesquioxanes oligomères polyédriques), la huntite, l'hydromagnésite ou la halloysite, ainsi que Sb₂O₃, Sb₂O₅, MoO₃, le stannate de zinc, l'hydroxystannate de zinc,
b) les retardateurs de flamme azotés tels que par exemple la mélamine, le mélem, le mélam, le mélon, les dérivés de la mélamine, les produits de condensation de la mélamine ou les sels de mélamine, la benzoguanamine, les polyisocyanurates, l'allantoïne, les phosphacènes, en particulier le cyanurate de mélamine, le phosphate de mélamine, le phosphate de dimélamine, le pyrophosphate de mélamine, le polyphosphate de mélamine, les phosphates de mélamine et de métaux tels que par exemple le phosphate de mélamine-aluminium, le phosphate de mélamine-zinc, le phosphate de mélamine-magnésium, ainsi que les pyrophosphates et les polyphosphates correspondants, la poly[2,4-(pipérazin-1,4-yl)-6-(morpholin-4-yl)-1,3,5-triazine], le polyphosphate d'ammonium, le borate de mélamine, le bromhydrate de mélamine,
c) les générateurs de radicaux,
d) les retardateurs de flamme phosphorés, tels que par exemple le phosphore rouge, les phosphates tels que par exemple le diphosphate de résorcinol, le diphosphate de bisphénol A et leurs oligomères, le phosphate de triphényle, le diphosphate d'éthylènediamine, les phosphinates tels que par exemple les sels de l'acide hypophosphoreux et ses dérivés tels que le phosphinate de diéthylaluminium ou le phosphinate d'aluminium, le phosphite d'aluminium, le phosphonate d'aluminium, les esters de phosphonates, les dérivés oligomères et polymères de l'acide méthanephosphonique, l'oxyde de 9,10-dihydro-9-oxa-10-phosphorylphénanthrène-10 (DOPO) et ses composés substitués,
e) les retardateurs de flamme halogénés à base de chlore et de brome, tels que par exemple les oxydes de diphényle polybromés, tels que par exemple l'oxyde de décabromodiphényle, le phosphate de tris(3-bromo-2,2-bis(bromométhyl)propyle, le phosphate de tris(tribromonéopentyle), l'acide tétrabromophtalique, le 1,2-bis(tribromophénoxy)éthane, l'hexabromocyclododécane, le diphényléthane bromé, l'isocyanurate de tris(2,3-dibromopropyle), l'éthylène-bis(tétrabromophtalimide), le tétrabromo-bisphénol A, le polystyrène bromé, le polybutadiène ou le polystyrène bromé, les copolymères de polybutadiène bromé, une résine époxyde bromée, le poly(acrylate de pentabromobenzyle), éventuellement en combinaison avec du Sb₂O₃ et/ou du Sb₂O₅,
f) les borates, tels que par exemple le borate de zinc ou le borate de calcium,
g) les agents anti-goutte, tels que par exemple le polytétrafluoréthylène,
h) les composés contenant du silicium, tels que par exemple les polyphénylsiloxanes, ainsi que les combinaisons et mélanges de ceux-ci.

8. Utilisation selon la revendication précédente, **caractérisée en ce que** les générateurs de radicaux sont choisis dans le groupe consistant en les N-alcoxyamines, les générateurs de radicaux -C-C-, les générateurs de radicaux comportant des groupes azo (-N=N-), les générateurs de radicaux comportant des groupes hydrazine (-NH-HN-), les générateurs de radicaux comportant des groupes hydrazone (>C=N-NH-), les générateurs de radicaux comportant des groupes azine (>C=N-N=C<), les générateurs de radicaux comportant des groupes triazène (-N=N-N<), les générateurs de radicaux comportant des groupes disulfure ou polysulfure (-S-S-), les générateurs de radicaux comportant des groupes thiol (-S-H), le sulfure de thiurame, les dithiocarbamates, le mercaptobenzothiazole et les sulfénamides, les générateurs de radicaux étant de préférence choisis dans le groupe consistant en
a) les N-alcoxylamines selon la formule développée présentée ci-après 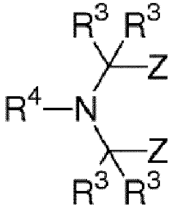 dans laquelle
R³ représente un atome d'hydrogène ou un radical alkyle, cycloalkyle, aryle, hétéroaryle ou acyle éventuellement substitué, en particulier un radical alkyle en C1 à C4,
R⁴ représente un radical alcoxy, aryloxy, cycloalcoxy, aralcoxy ou acyloxy,
Z représente un atome d'hydrogène ou un radical alkyle, cycloalkyle, aryle, hétéroaryle ou acyle éventuellement substitué, les deux radicaux Z pouvant aussi former un cycle fermé, qui peut éventuellement être substitué par des groupes ester, éther, amine, amide, carboxy ou uréthanne,
b) les composés azoïques selon les formules développées représentées ci-après : 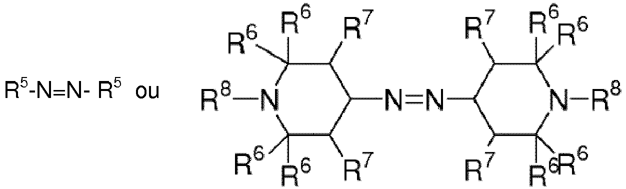 dans lesquelles
R⁵ est un radical alkyle, cycloalkyle ou aryle,
R⁶ à chaque occurrence, est identique ou différent, et représente un radical alkyle à chaîne droite ou ramifiée,
R⁷ à chaque occurrence, est identique ou différent, et représente un atome d'hydrogène ou un radical alkyle à chaîne droite ou ramifiée, et
R⁸ à chaque occurrence, est identique ou différent, et représente un radical alkyle, alcoxy, aryloxy, cycloalkyloxy, aralcoxy ou acyloxy,
c) les dicumylènes selon la formule développée représentée ci-après 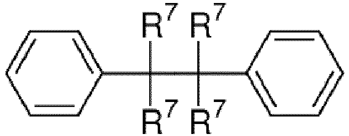 dans laquelle R⁷ a les significations données ci-dessus, de préférence représente le groupe méthyle,
d) et/ou les polycumylènes selon la formule développée représentée ci-après 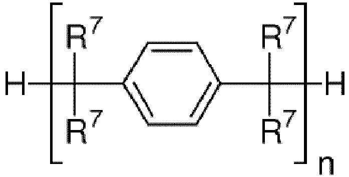 dans laquelle R⁷ a les significations données ci-dessus, de préférence représente le groupe méthyle, et 2 < n < 100.

9. Utilisation selon l'une des deux revendications précédentes, **caractérisée en ce que** les oxyimides organiques et le ou les retardateurs de flamme supplémentaires sont utilisés selon un rapport en poids de 99:1 à 1:99, de préférence de 5:95 à 50:50, d'une manière particulièrement préférée de 10:90 à 30:70.

10. Utilisation selon l'une des revendications précédentes, **caractérisée en ce que** les oxyimides organiques sont utilisés en des quantités, rapportées aux plastiques, de 0,01 à 30 % en poids, de préférence de 0,1 à 20 % en poids, d'une manière particulièrement préférée de 1 à 10 % en poids.

11. Composition de plastiques ignifugée, contenant
a) 50 à 98 parties en poids, de préférence 70 à 95 parties en poids, d'au moins un plastique, en particulier d'au moins un polymère thermoplastique,
b) 1 à 25 parties en poids, de préférence 2,5 à 15 parties en poids d'au moins un oxyimide organique contenant au moins un élément structural ayant la formule I représentée ci-après 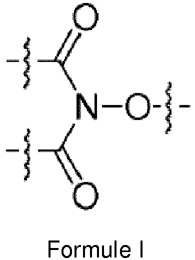 l'oxyimide organique étant non halogéné,
c) 1 à 25 parties en poids, de préférence 2,5 à 15 parties en poids, d'au moins un retardateur de flamme supplémentaire, et contenant en outre de préférence
d) jusqu'à 40 parties en poids d'au moins un agent de renforcement ou d'une charge, et/ou
e) jusqu'à 5 parties en poids d'au moins un additif de la classe des antioxydants phénoliques, des phosphines, des fixateurs d'acide, des amines à empêchement stérique, des dispersants ou des combinaisons de ceux-ci,
ou en étant constituée.

12. Composition de plastiques ignifugée selon l'une des deux revendications précédentes, **caractérisée en ce que** les additifs sont choisis dans le groupe consistant en les absorbants UV, les photostabilisants, les stabilisants, les hydroxylamines, les benzofurannones, les désactivateurs de métaux, les désactivateurs de charges, les agents de nucléation, les agents améliorant la résistance au choc, les plastifiants, les lubrifiants, les agents modifiant la rhéologie, les auxiliaires de mise en oeuvre, les pigments, les colorants, les azurants optiques, les matières actives antimicrobiennes, les antistatiques, les agents de glissement, les agents anti-adhérence de contact, les agents de couplage, les dispersants, les agents de compatibilisation, les fixateurs d'oxygène, les fixateurs d'acides, les agents de marquage ou les agents antivoile.

13. Procédé de fabrication d'une composition de plastiques ignifugée selon l'une des revendications 11 à 12, dans lequel on introduit
a) 1 à 25 parties en poids, de préférence 2,5 à 15 parties en poids d'au moins un oxyimide organique, contenant au moins un élément structural ayant la formule I représentée ci-après 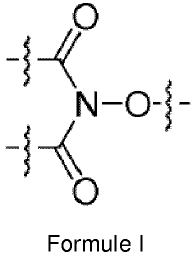 l'oxyimide organique étant non halogéné,
b) avant, après ou en même temps, 1 à 25 parties en poids, de préférence 2,5 à 15 parties en poids d'au moins un retardateur de flamme supplémentaire,
dans 50 à 98 parties en poids, de préférence 70 à 95 parties en poids d'au moins un plastique, en particulier d'au moins un polymère thermoplastique.

14. Mélange à mouler, pièce moulée, vernis ou revêtement, pouvant être fabriqué à partir d'une composition de plastiques ignifugée selon l'une des revendications 1 à 12, en particulier sous forme de pièces moulées par injection, de feuilles ou de films, de revêtements ou de vernis, de mousses, de fibres, de câbles et de tubes, de profilés, de bandelettes, de membranes, telles que par exemple les géomembranes, d'adhésifs, qui sont fabriqués par extrusion, moulage par injection, moulage par soufflage, calandrage, pressage, filage, ou par des procédés d'enduction et de revêtement, par exemple pour l'industrie électrique et électronique, l'industrie du bâtiment, l'industrie des transports, pour les appareils ménagers et électriques, pour les éléments de véhicules, pour les articles de grande consommation, les meubles, les textiles.

15. Composition retardatrice de flamme consistant en un oxyimide organique contenant au moins un élément structural ayant la formule I représentée ci-après 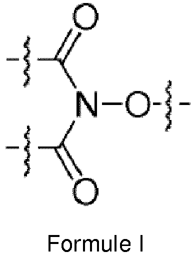 et au moins un retardateur de flamme supplémentaire, l'oxyimide organique étant non halogéné.
